(19) Europäisches Patentamt
European Patent Office
Office européen des brevets

(11) EP 1 410 786 B1

(12) # FASCICULE DE BREVET EUROPEEN

(45) Date de publication et mention de la délivrance du brevet:
**02.01.2008 Bulletin 2008/01**

(51) Int Cl.:
***A61Q 1/02*** *(2006.01)* ***A61K 8/49*** *(2006.01)*

(21) Numéro de dépôt: **03292570.3**

(22) Date de dépôt: **15.10.2003**

(54) **Composition cosmétique associant au moins deux matières colorantes dont au moins une matière colorante photochrome**

Kosmetische Zusammensetzung enthaltend mindestens zwei Farbstoffe wobei mindestens einer derselben Photochromizität aufweist

Cosmetic composition comprising at least two dyes whereof at least one is photochromic

(84) Etats contractants désignés:
**AT BE BG CH CY CZ DE DK EE ES FI FR GB GR HU IE IT LI LU MC NL PT RO SE SI SK TR**

(30) Priorité: **18.10.2002 FR 0213038**
**18.10.2002 FR 0213036**

(43) Date de publication de la demande:
**21.04.2004 Bulletin 2004/17**

(73) Titulaire: **L'ORÉAL**
**75008 Paris (FR)**

(72) Inventeurs:
- **Simon, Jean-Christophe**
**162-0842 Tokyo (JP)**
- **Blin, Xavier**
**75015 Paris (FR)**

(74) Mandataire: **Le Coupanec, Pascale A.M.P.**
**Nony & Associés,**
**3 rue de Penthièvre**
**75008 Paris (FR)**

(56) Documents cités:
**EP-A- 0 970 689**
**EP-B1- 0 973 761**
**WO-A-02/078665**
**FR-A- 2 815 848**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à une composition cosmétique, notamment de maquillage de la peau, des lèvres et/ou des phanères associant au moins deux matières colorantes dont au moins une matière colorante photochrome.

Par composition cosmétique, on désigne une composition telle que définie dans la directive 93/35/CEE du conseil du 14 juin 1993.

La composition selon l'invention est utilisable notamment dans le domaine du maquillage ou du soin des matières kératiniques comme la peau, les cils, les sourcils, les cheveux et les ongles d'êtres humains. En l'occurrence, elle peut se présenter sous forme de mascara, de produit pour les sourcils, d'eye-liner, de fard à paupières, de fard à joues, de fond de teint, de produit pour les lèvres, de produit de maquillage du corps (tatouage semi-permanent), de produit de maquillage des cheveux.

L'une des fonctions principales du maquillage consiste à apporter des effets de couleurs et/ou optiques au niveau des zones à maquiller sur la peau, les lèvres, les cils et les ongles.

D'une manière générale, ces effets sont statiques, c'est-à-dire n'évoluent ni au cours du temps, ni en fonction de la luminosité ambiante. L'évolution la plus fréquente consiste généralement en une altération progressive dans le temps des effets de couleurs et/ou optique d'origine. Cette altération est généralement due soit à un transfert et/ou une migration de la composition, phénomènes particulièrement prononcés pour les rouges à lèvres, soit à une mauvaise tenue des pigments présents dans la composition de maquillage vis-à-vis du sébum, phénomène plus particulièrement rencontré avec les fonds de teint.

Il n'existe donc pas à ce jour de composition de maquillage que l'on pourrait qualifier de dynamique en terme de teinte, c'est-à-dire susceptible de se modifier significativement en terme de couleur et/ou d'éclat, en réponse par exemple à une modification de la luminosité ambiante.

Or, il est clair que ce type de composition de maquillage dotée d'un effet couleur et/ou optique dynamique répondrait précisément à un souhait des consommatrices qui sont à la recherche de maquillages colorés originaux contrastant avec des compositions de maquillage conventionnelles.

L'invention a précisément pour objectif principal de répondre à ce besoin.

En l'occurrence, la présente invention met notamment à profit la faculté manifestée par certaines matières colorantes photochromes à changer de couleur dès qu'elles sont soumises à un rayonnement contenant des rayons ultraviolets et ceci d'une manière réversible.

L'utilisation de pigments photochromes dans des compositions cosmétiques est connue. La demande EP 970 689 décrit un produit bi-couche de maquillage permettant de superposer à une couche de base contenant un pigment photochrome, une couche de surface contenant un filtre de lumière ultraviolette. Le maquillage correspondant permet de former des motifs qui apparaissent ou disparaissent selon la nature de la lumière mais il ne lui est associé aucune dynamique en terme d'effet de couleur. La demande WO 02/078665 propose pour sa part des compositions cosmétiques comprenant des agents photochromes de type naphtopyrane. Toutefois, ceux-ci sont incorporés dans la composition cosmétique sous une forme liquide encapsulée dans une microcapsule. Cette forme dispersible est notamment décrite comme dotée d'une activité anti-oxydante.

D'une manière inattendue, les inventeurs ont constaté qu'il était particulièrement avantageux d'associer au moins une matière colorante conventionnelle à au moins une matière colorante photochrome spécifique pour obtenir un effet dynamique original en terme de couleur et d'éclat.

Plus précisément, la présente invention concerne selon un de ses aspects, une composition cosmétique comprenant dans un milieu physiologiquement acceptable au moins deux matières colorantes, dont au moins une matière colorante photochrome, caractérisée en ce que ladite matière colorante photochrome présente un écart de teinte $\Delta E$ au moins égal à 5 et est au moins un dérivé naphtopyrane présent dans la composition sous une forme solubilisée et de type 2H-naphto-[2,1-b]-pyrane de formule (I) ou 3H-naphto-[2,1-b]-pyrane de formule (II) tels que définis ci-après.

La présente invention vise également selon un autre de ses aspects, l'utilisation d'au moins une matière colorante photochrome conforme à l'invention dans une composition cosmétique, notamment pour le maquillage de la peau, des lèvres et/ou des phanères, contenant au moins une seconde matière colorante.

La présente invention concerne encore selon un autre de ses aspects, un procédé de maquillage de la peau, des lèvres et/ou des phanères comprenant l'application sur la peau, les lèvres et/ou les phanères d'au moins une composition conforme à l'invention.

Selon une première variante, la composition selon l'invention associe ladite matière colorante photochrome à au moins une seconde matière colorante, non photochrome au sens de l'invention et notamment choisie parmi les agents de coloration goniochromatiques, colorants hydrosolubles ou liposolubles, pigments, particules réfléchissantes et/ou nacres.

Selon une seconde variante, la composition cosmétique comprend au moins deux matières colorantes photochromes distinctes.

Les inventeurs ont ainsi constaté que l'association d'au moins une matière colorante photochrome conforme à l'invention à au moins une seconde matière colorante plus conventionnelle et notamment non photochrome dans une composition cosmétique, notamment de maquillage permettait de conférer à celle-ci une dynamique rapide et réversible en terme d'effet de teinte et/ou d'éclat.

L'écart de teinte est suffisamment significatif pour être remarqué à l'oeil nu.

Il est immédiat, c'est-à-dire se manifeste dans un délai très court, après soit exposition de la composition maquillage à un rayonnement UV, soit arrêt de cette exposition.

Outre cet aspect « effet de teinte et/ou d'éclat dynamique », les compositions selon l'invention sont tout particulièrement avantageuses pour suppléer au phénomène d'altération des couleurs qui a été discuté précédemment et qui est généralement lié à une mauvaise tenue des pigments conventionnels au sébum. L'association à ces pigments d'une matière colorante photochrome conforme à l'invention permet en effet avantageusement de compenser ce type d'altération dans la mesure où cette matière colorante photochrome est capable à elle seule, en réponse à une excitation lumineuse de type UV, de restituer un effet coloré pouvant être équivalent à celui procuré initialement par lesdits pigments.

## MATIERE COLORANTE PHOTOCHROME

D'une manière générale, une matière colorante photochrome est une matière colorante ayant la propriété de changer de teinte lorsqu'elle est éclairée par de la lumière ultraviolette et de rétablir sa couleur initiale lorsqu'elle n'est plus éclairée par cette lumière ou encore de passer d'un état non coloré à un état coloré et inversement. En d'autres termes, une telle matière présente des teintes différentes selon qu'elle est éclairée par de la lumière contenant un certaine quantité de radiations UV comme la lumière solaire ou de la lumière artificielle.

Au sens de la présente invention, la matière colorante photochrome présente un écart de teinte $\Delta E$ au moins égal à 5.

Au sens de la présente invention, $\Delta E$ figure l'écart de teinte observé au niveau de la matière photochromique entre son état excité, c'est-à-dire en présence d'irradiation UV et son état non excité, c'est-à-dire en absence d'irradiation UV.

Le $\Delta E$ peut être déterminé à partir de l'espace chromatique et plus particulièrement à partir des coordonnées chromatiques spécifiques de la matière colorante photochrome considérée, évaluées d'une part au terme de 2 minutes d'exposition à une radiation UV, généralement à l'aide d'une lumière artificielle contenant un rayonnement UV, et d'autre part au repos, c'est-à-dire 5 secondes après l'arrêt de la radiation.

Plus précisément, le protocole de mesure est le suivant :

- 1 % en poids de la matière colorante photochrome considérée est formulée dans 100 % en poids d'une base blanche de rouge à lèvres de la composition suivante :

- octyl-2 dodecanol 0,5 %
- hectorite modifiée par chlorure de di-stearyl di-methyl ammonium 0,6 %
- lanoline liquide 27,2 %
- cire microcristalline 10,5 %
- cire d'abeille polyglycérolée (3 moles) 4,2 %
- lanoline acétylée 6,7 %
- huile d'arara (esters d'acide oléique) 13,5 %
- cire de lanoline oxypropylénée (5 op) 6,7 %
- érucate d'oléyle 13,5 %
- triglycérides d'acides oléique-linoléique-linolénique 1,7 %
- triglycérides d'acides palmitique-oléique-linoléique 13,5 %
- hyaluronate de sodium 0,1 %
- conservateurs 0,1 %
- vitamine 0,5 %
- filtre UV 0,7 %

Sur une carte de contraste de marque LENETA et de référence FORM 1A PENOPAC, on étale une couche de 50 $\mu$m d'épaisseur de la composition, à l'aide d'un étaleur automatique. La couche recouvre au moins le fond blanc de la carte.

Les mesures de réflexion sont effectuées à l'aide d'un spectrocolorimètre MINOLTA 3700D. On détermine ainsi les coordonnées trichromatiques initiales de la composition avant irradiation ($L_o$, $a_o$ et $b_o$). On soumet ensuite la composition à un flux de 2 $mW/cm^2$ d'un rayonnement UVA pendant deux minutes puis aussitôt après l'arrêt de l'irradiation, on détermine les nouvelles coordonnées trichromatiques (L, a et b). Il s'écoule moins de 5 secondes entre l'arrêt

de l'irradiation et la détermination des nouvelles coordonnées.

Le $\Delta E$ est calculé de la manière suivante :

$$\Delta E = [(L - L_o)^2 + (a - a_o)^2 + (b - b_o)^2]^{1/2}$$

Avantageusement, les matières colorantes photochromes selon l'invention possèdent un $\Delta E$ supérieur ou égal à 10, en particulier supérieur ou égal à 25 plus particulièrement supérieur ou égal à 35 voire supérieur ou égal à 45.

Une mesure de $\Delta E$ supérieure à 30 environ signifie que la matière colorante photochrome donne une couleur vive.

Dans le cadre de la présente invention, les matières colorantes photochromes ont par ailleurs pour avantage de répondre à bref délai soit à une excitation par de la lumière UV soit à un arrêt d'une telle excitation. Il s'en suit une modification rapide en terme de coloration. Cette modification de teinte peut ainsi se traduire dans un laps de temps avantageusement inférieur ou égal à 2 minutes, notamment inférieur ou égal à 1 minute et en particulier inférieur ou égal à 50 secondes.

Selon une variante de l'invention, la composition revendiquée contient au moins deux matières colorantes photochromes conformes à l'invention, en association ou non à une autre matière colorante notamment non photochrome.

La matière colorante photochrome considérée selon l'invention est généralement présente en une quantité efficace pour conférer à ladite composition un effet dynamique en terme de couleur et d'éclat. Cette quantité peut varier de 0,001 à 20 % en poids, notamment de 0,005 à 10 % en poids, en particulier de 0,01 à 5 % en poids, et plus particulièrement de 0,05 à 2 % en poids, voire de 0,1 à 1 % en poids, par rapport au poids total de la composition cosmétique.

Plus précisément, la matière colorante organique photochrome selon l'invention est de type naphtopyrane, et choisie parmi des 3H-naphto-[2,1-b]-pyranes qui peuvent être représentés par la formule (I) et des 2H-naphto-[1,2-b]-pyranes qui peuvent être représentés par la formule (II) :

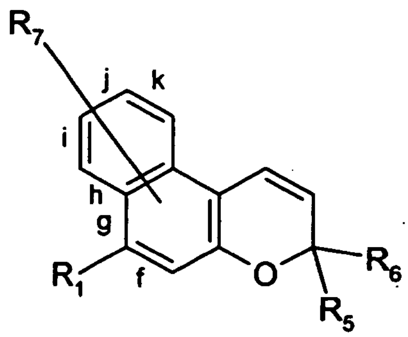

(I)

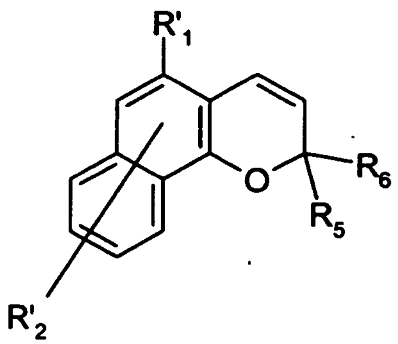

(II)

dans lesquelles :

- R1 représente :
  - (i) un atome d'hydrogène;
  - (ii) un groupement hydrocarboné ayant 1 à 30, notamment 1 à 18, et en particulier 1 à 12, voire 1 à 6 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (iii) un cycle hydrocarboné formé avec l'une des liaisons "f" ou "gh" et le radical $R_7$; ou
  - (iv) un groupement choisi parmi $-COOR_4$, $-C(O)NR_2R_3$, $-NR_2R_3$, $-OR_4$ et $-SR_4$, dans lequel :

- $R_2$ et $R_3$ soit représentent, indépendamment l'un de l'autre, un groupement hydrocarboné ayant 1 à 20, notamment 1 à 12, et en particulier 1 à 6 atomes de carbone, linéaire, ramifié ou cyclique, saturé ou insaturé, éventuellement comportant 1 à 5 hétéroatomes choisis parmi N, O, S, Si et P,
  soit pris ensemble avec l'atome d'azote auquel ils sont reliés, forment un hétérocycle hydrocarboné, saturé ou

insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P;

- $R_4$ représente un groupement hydrocarboné ayant 1 à 20, notamment 1 à 12, et en particulier 1 à 6 atomes de carbone, linéaire, ramifié ou cyclique, saturé ou insaturé, éventuellement halogéné ou perhalogéné, notamment par F, Br et/ou Cl, et/ou éventuellement comportant 1 à 5 hétéroatomes choisis parmi N, O, S, Si et P;
- $R_5$ et $R_6$ représentent, indépendamment l'un de l'autre, un groupement choisi parmi :

  - (i) les groupements aminoaryles cycliques saturés de formule (IIA) ou (IIB) :

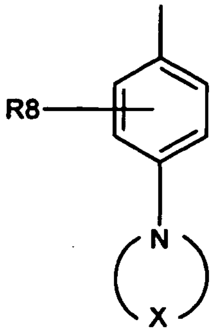

(IIA)

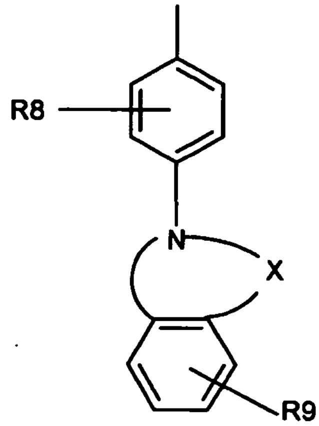

(IIB)

dans lesquelles le cycle comportant N et X est un cycle saturé qui comprend au total 3 à 30 atomes, notamment 4 à 10 et en particulier 5 à 8, voire 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;

  - (ii) les groupements indolinoaryles de formule (III) :

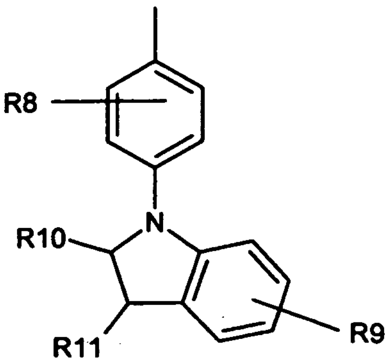

(III)

dans laquelle $R_{10}$ et $R_{11}$ représentent, indépendamment l'un de l'autre, un groupement choisi parmi (i) les groupements hydrocarbonés ayant 1 à 30, notamment 1 à 18, et en particulier 1 à 12, voire 1 à 6 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl; (ii) les atomes d'halogène, et notamment F, Br et/ou Cl; (iii) les groupements -CN (nitrile), -COOH (carboxylate),

$-NO_2$ (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus; (vi) les radicaux $R_{10}$ et $R_{11}$ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, voire 1 à 12 atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

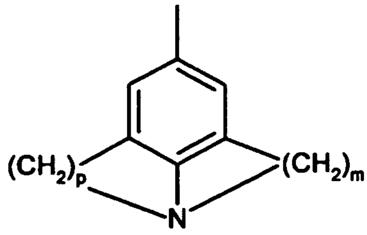

(IV)

dans laquelle m et p sont, indépendamment l'un de l'autre, des entiers allant de 2 à 5;

- (iv) les groupements aminoaryles cycliques insaturés de formules (VA), (VB) ou (VC) :

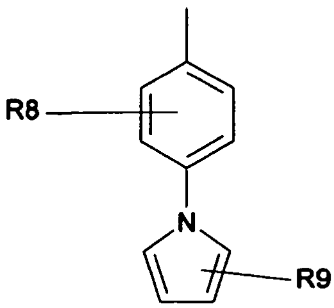

(VA)

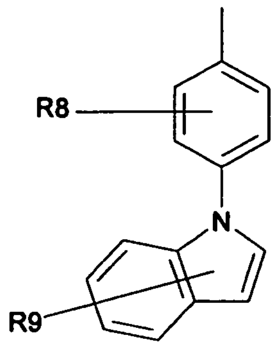

(VB)

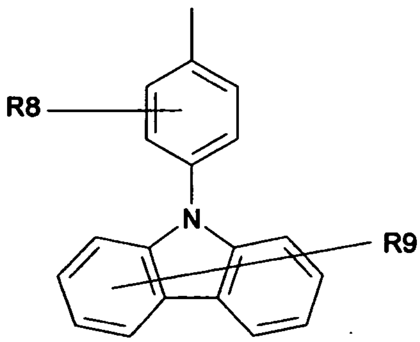

(VC)

dans lesquelles $R_8$ et $R_9$, représentent, indépendamment l'un de l'autre, un groupement choisi parmi (i) les groupements hydrocarbonés ayant 1 à 30, notamment 1 à 18, et en particulier 1 à 12, voire 1-6 atomes de

carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl; (ii) les atomes d'halogène, et notamment F, Br et/ou Cl; (iii) les groupements -CN (nitrile), -COOH (carboxylate), $-NO_2$ (nitro); (iv) un atome d'hydrogène; (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;

- (v) un groupement hydrocarboné ayant 1 à 30, notamment 2 à 18, et en particulier 3 à 12 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

- $R_7$ représente un groupement choisi parmi :

  - (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (ii) les atomes d'halogène, et notamment F, Br et/ou Cl;
  - (iii) les groupements -CN (nitrile), -COOH (carboxylate), $-NO_2$ (nitro); -N=N- (azo); =NH (imino); $-CONH_2$(amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
  - (vi) le radical $R_7$ pouvant en outre former, avec l'une des liaisons "i", "j", "k", ou "g,h" prises avec le radical $R_1$, ou "f" prise avec le radical $R_1$, un cycle hydrocarboné saturé ayant au total 3 à 8, notamment 4 à 7, et en particulier 5 ou 6, atomes de carbone, éventuellement comportant 1 à 5 hétéroatomes choisis parmi N, O, S, Si et P;

- $R'_1$ représente un groupement choisi parmi :

  - un atome d'hydrogène;
  - (ii) un groupement hydrocarboné ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (iii) un groupement choisi parmi $-C(O)NR_2R_3$, $-NR_2R_3$, $-OR_4$ et $-SR_4$, avec $R_2$, $R_3$ et $R_4$ ayant les significations données ci-dessus;

- $R'_2$ représente un groupement choisi parmi :

  - (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
  - (ii) les atomes d'halogène, et notamment F, Br et/ou Cl;
  - (iii) les groupements -CN (nitrile), -COOH (carboxylate), $-NO_2$(nitro); -N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus.

Notamment, les matières colorantes organiques photochromes selon l'invention peuvent répondre à l'une des formules (Ia) et (IIa) suivantes :

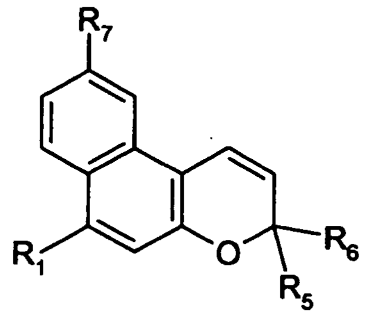

(Ia)

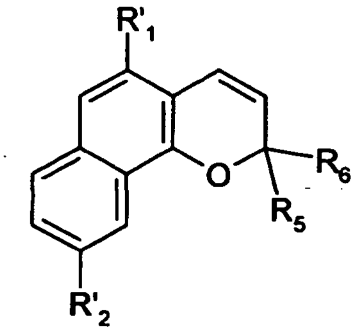

(IIa)

dans lesquelles $R_1$, $R_5$, $R_6$, $R_7$, $R'_1$ et $R'_2$ sont définis tels que précédemment.

En particulier, $R_1$ peut représenter un atome d'hydrogène; un cycle hydrocarboné avec l'une des liaisons "f" ou "gh" et le radical $R_7$; ou un groupement choisi parmi $-COOR_4$, $-NR_2R_3$, $-OR_4$ et $-SR_4$, dans lequel :

- $R_2$ et $R_3$ soit peuvent représenter, indépendamment l'un de l'autre, un groupement hydrocarboné ayant 1 à 20, notamment 1 à 12, et en particulier 1 à 6 atomes de carbone, linéaire, ramifié ou cyclique, saturé ou insaturé, éventuellement comportant 1 à 5 hétéroatomes choisis parmi N, O, S, Si et P,
  soit pris ensemble avec l'atome d'azote auquel ils sont reliés, peuvent former un hétérocycle hydrocarboné, saturé ou insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P;
- $R_4$ peut représenter un groupement hydrocarboné ayant 1 à 20, notamment 1 à 15, et en particulier 1 à 6 atomes de carbone, linéaire, ramifié ou cyclique, saturé ou insaturé, éventuellement halogéné ou perhalogéné, notamment par F, Br et/ou Cl, et/ou éventuellement comportant 1 à 5 hétéroatomes choisis parmi N, O, S, Si et P.

En l'occurrence $R_5$ et $R_6$ peuvent représenter, indépendamment l'un de l'autre, un groupement choisi parmi :

- les groupements aminoaryles cycliques saturés de formule (IIA) ou (IIB) :

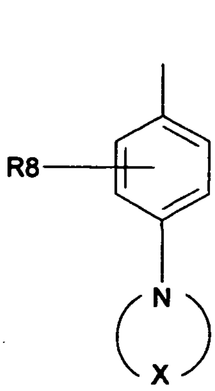

(IIA)

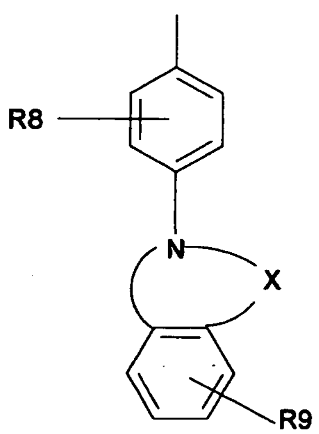

(IIB)

dans lesquelles le cycle comportant N et X est un cycle saturé qui comprend au total 3 à 30 atomes, notamment 4 à 10 et encore en particulier 5, 6 ou 7 atomes, inclus l'azote, le reste étant des atomes de carbone et/ou des

hétéroatomes choisis parmi O, S, Si, P et/ou des groupements choisis parmi -NH et -NR avec R représentant un radical hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;

- un groupement hydrocarboné ayant 1 à 30, notamment 2 à 18, et en particulier 3 à 12 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

Notamment, $R_7$ peut représenter un groupement choisi parmi :

- (i) les groupements hydrocarbonés ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl;
- (ii) les atomes d'halogène, et notamment F, Br et/ou Cl;
- (iii) les groupements -CN (nitrile), -COOH (carboxylate), $-NO_2$ (nitro); -N=N- (azo); =NH (imino); $-CONH_2$ (amide);
- (iv) un atome d'hydrogène;
- (v) un groupement choisi parmi $-NR_2R_3$, $-OR_4$ ou $-SR_4$ avec $R_2$, $R_3$ et $R_4$ ayant les significations données ci-dessus;
- (vi) le radical $R_7$ pouvant en outre former, avec l'une des liaisons "i", "j ", "k", ou "g,h" prises avec le radical $R_1$, ou "f" prise avec le radical $R_1$, un cycle hydrocarboné saturé ayant au total 3 à 8, notamment 4 à 7, et en particulier 5 ou 6 atomes de carbone, éventuellement comportant 1 à 5 hétéroatomes choisis parmi N, O, S, Si et P.

En particulier, $R'_1$ peut représenter l'hydrogène ou un groupement hydrocarboné ayant 1 à 30, notamment 1 à 18, et en particulier 1 à 12 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par F, Br et/ou Cl.

Notamment, $R'_2$ représente l'hydrogène ou un groupement choisi parmi $-NO_2$, $-NR_2R_3$ et $-C(O)NR_2R_3$, dans lesquels $R_2$ et $R_3$, soit représentent, indépendamment l'un de l'autre, un groupement hydrocarboné ayant 1 à 20, notamment 1 à 12, et en particulier 1 à 6, atomes de carbone, linéaire, ramifié ou cyclique, saturé ou insaturé, éventuellement comportant 1 à 5 hétéroatomes choisis parmi N, O, S, Si et P; soit pris ensemble avec l'atome d'azote auquel ils sont reliés, forment un hétérocycle hydrocarboné, saturé ou insaturé, comportant 3 à 10, notamment 4 à 6 atomes de carbone et éventuellement 1 à 5 autres hétéroatomes choisis parmi N, O, S, Si et P, ledit cycle étant éventuellement substitué par au moins un radical hydrocarboné ayant 1 à 20, notamment 1 à 15, et en particulier 1 à 6 atomes de carbone, linéaire, ramifié ou cyclique, saturé ou insaturé, comportant éventuellement 1 à 5 hétéroatomes choisis parmi N, O, S, Si et P.

On peut plus particulièrement citer les colorants organiques de formule (I) ou (Ia) pour lesquelles :

- $R_1$ représente l'hydrogène; ou un groupement -COOR avec R étant un radical hydrocarboné saturé ayant 1 à 12, notamment 1 à 6 atomes de carbone, et notamment un radical méthyle ou éthyle; ou un groupement

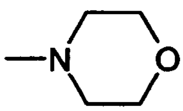

et/ou

- $R_5$ et $R_6$ représentent, indépendamment l'un de l'autre, soit (i) un groupement de formule (IIA) :

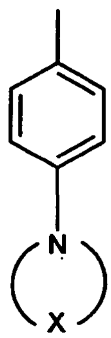

(IIA)

dans laquelle le cycle comportant N et X est un cycle saturé qui comprend au total 4 à 7 atomes, notamment 5 ou 6 atomes, inclus l'azote, et notamment 3 à 5 atomes de carbone et 0 ou 1 atome d'oxygène; et en particulier un groupement de formule:

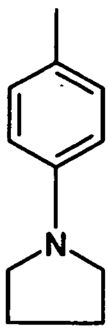

ou

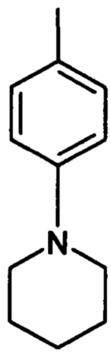

ou

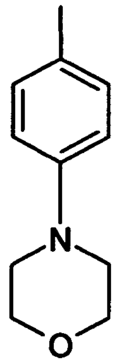

soit (ii) un groupement hydrocarboné ayant 5 à 14, notamment 6 à 10 atomes de carbone, linéaire, ramifié ou cyclique, saturé ou insaturé, éventuellement comportant 1 à 2 hétéroatomes choisis parmi N, O ou S;
en particulier un groupement

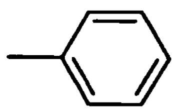

ou

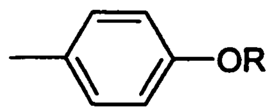

ou

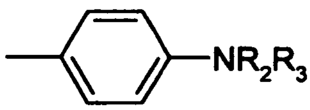

dans lesquelles R est un radical hydrocarboné saturé ayant 1 à 12, notamment 1 à 6 atomes de carbone, et notamment un radical méthyle ou éthyle; et $R_2$ et $R_3$ sont, indépendamment l'un de l'autre, un groupement hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;
et/ou

- $R_7$ représente un atome d'hydrogène ou un groupement $-NR_2R_3$, avec $R_2$ et $R_3$ représentant, indépendamment l'un de l'autre, un groupement hydrocarboné saturé, linéaire ou ramifié, ayant 1 à 12, notamment 1 à 6 atomes de carbone, et notamment un groupement méthyle et/ou éthyle.

On peut également citer les matières colorantes photochromes de formule (II) ou (IIa) pour lesquelles :

- $R'_1$ représente l'hydrogène ou un groupement -COOR avec R étant un radical hydrocarboné saturé ayant 1 à 12, notamment 1 à 6 atomes de carbone, et notamment un radical méthyle ou éthyle;
et/ou
- $R_5$ et $R_6$ représentent, indépendamment l'un de l'autre, soit (i) un groupement de formule (IIA) :

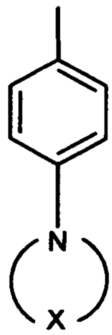

(IIA)

dans laquelle le cycle comportant N et X est un cycle saturé qui comprend au total 4 à 7 atomes, notamment 5 à 6 atomes, inclus l'azote, et notamment 4 à 5 atomes de carbone et 0 ou 1 atome d'oxygène; et en particulier un groupement de formule:

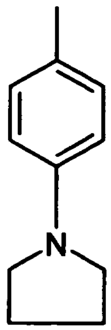

ou

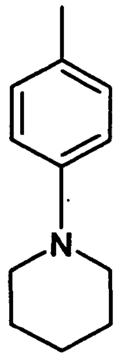

ou

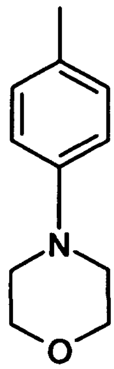

soit (ii) un groupement hydrocarboné ayant 5 à 14, notamment 6 à 10 atomes de carbone, linéaire, ramifié ou cyclique, saturé ou insaturé, éventuellement comportant 1 à 2 hétéroatomes choisis parmi N, O ou S;
en particulier un groupement :

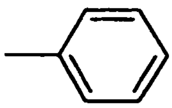

ou

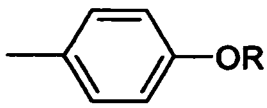

ou

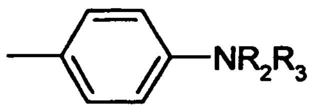

dans lesquelles R est un radical hydrocarboné saturé ayant 1 à 12, notamment 1-6 atomes de carbone, et notamment un radical méthyle ou éthyle; et $R_2$ et $R_3$ sont, indépendamment l'un de l'autre, un groupement hydrocarboné ayant 1 à 20, notamment 1 à 15, et en particulier 1 à 6 atomes de carbone, linéaire, ramifié ou cyclique, saturé ou insaturé, éventuellement comportant 1 à 5 hétéroatomes choisis parmi N, O, S, Si et P;
et/ou

- R'$_2$ représente l'hydrogène, ou un groupement -NR'R" avec R' et R", identiques ou différents, représentant un groupement hydrocarboné saturé, linéaire ou ramifié, ayant 1 à 12, notamment 1 à 6 atomes de carbone, et notamment un groupement méthyle et/ou éthyle; ou un groupement

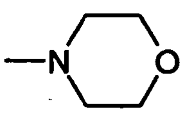

A titre représentatif et non limitatif des matières colorantes de type naphtopyrane, on peut citer celles décrites dans les demandes WO94/22850, WO98/45281 et WO00/18755.

Conviennent tout particulièrement à l'invention les composés suivants :

Le 3,3-di(4-méthoxyphényl)-6-morpholino-3H-naphto[2,1-b]pyrane de formule :

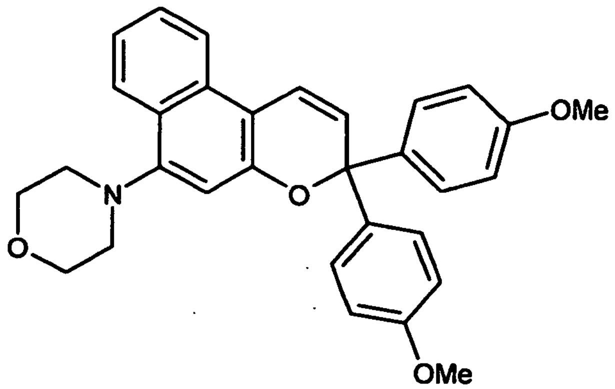

Le 3-phényl-3-(4-morpholinophényl)-6-morpholino-3H-naphto[2,1-b]pyrane de formule :

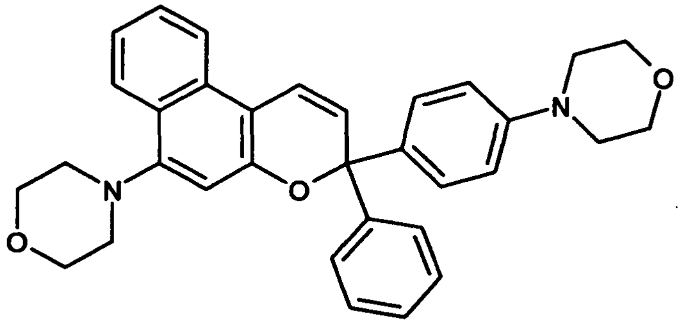

Le 3-phényl-3-(4-pipéridinophényl)-6-morpholino-3H-naphto[2,1-b]pyrane de formule :

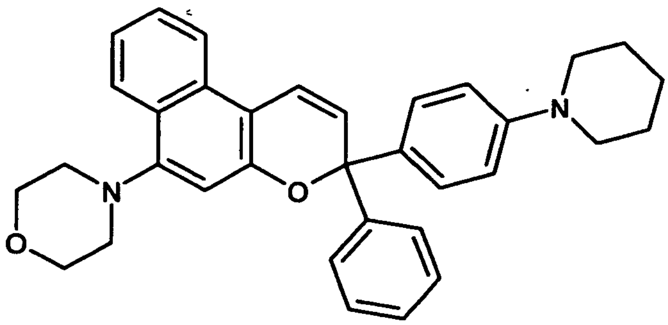

Le 3-phényl-3-(4-pipéridinophényl)-6-carboxyméthyl-9-N-diméthyl-3H-naphto[2,1-b]pyrane de formule :

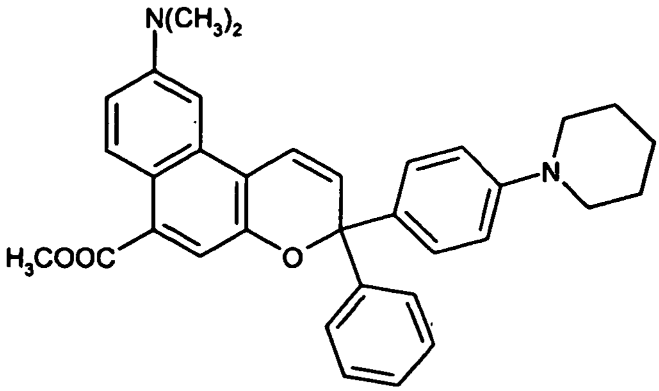

Le 2-phényl-2-(4-piperidinophényl)-5-carboxyméthyl-9-N-diméthyl-2H-naphto[1,2-b]pyrane de formule :

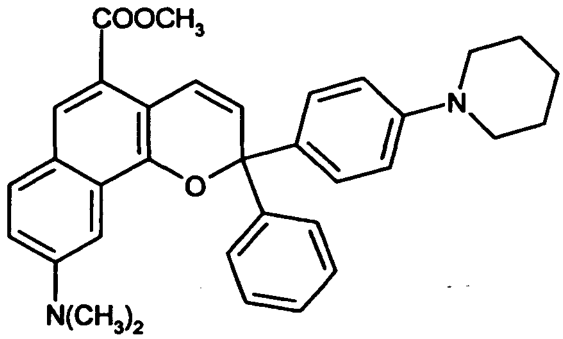

et leurs mélanges.

Le dérivé naphtopyrane conforme à l'invention est présent sous une forme solubilisée au sein de la composition selon l'invention. En conséquence, le choix des matières colorantes photochromes susceptibles d'être mis en oeuvre dans le cadre de la présente invention est effectué en prenant en compte leur solubilité dans la phase ou l'une des phases composant le milieu physiologiquement acceptable de la composition selon l'invention.

## AUTRES MATIÈRES COLORANTES

Au sens de la présente invention, on entend couvrir sous le terme « autres matières colorantes » ou « seconde matière colorante », des matières colorantes non photochromes au sens de la présente invention et notamment tout composé choisi parmi les colorants monochromes liposolubles et hydrosolubles, les nacres, les particules réfléchissantes et les agents de coloration goniochromatiques.

### *Les agents de coloration goniochromatiques*

Par « agent de coloration goniochromatique », on désigne au sens de la présente invention un agent de coloration permettant d'obtenir, lorsque la composition cosmétique est étalée sur un support, un trajet de couleur dans le plan a*b* de l'espace colorimétrique CIE 1976 qui correspond à une variation Dh de l'angle de la teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition cosmétique ait été étalée à l'état fluide avec une épaisseur de 300 $\mu$m au moyen d'un étaleur automatique sur une carte de contraste de marque ERICHSEN et de référence Typ 24/5, la mesure étant effectuée sur le fond noir de la carte.

L'agent de coloration goniochromatique peut être présent par exemple en une quantité pouvant varier, en poids par rapport au poids total de la composition, de 0,1 à 60 %, voire de 1 à 20 % ou de 2 à 15 %, et mieux de 2 à 10 % en poids, notamment pour une composition destinée à être appliquée sur les lèvres. Une composition de vernis à ongles

pourra contenir par exemple entre 0,1 % et 5 % d'agent de coloration goniochromatique ; un fond de teint pourra en contenir de 10 à 15 % et un rouge à lèvres pourra en contenir de 2 à 8 % en poids.

L'agent de coloration goniochromatique peut être choisi de manière à présenter un changement de couleur relativement important avec l'angle d'observation.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

Quant aux particules goniochromatiques à cristaux liquides susceptibles d'être mises en oeuvre dans la composition selon l'invention, elles peuvent notamment être à base de polymère susceptible d'être obtenu par polymérisation d'un mélange de monomères comprenant :

- a) au moins un premier monomère A de formule (I) Y1-A1-M1-A2-Y2 dans laquelle

  - i) Y1 et Y2 , identiques ou différents, représentent un groupe polymérisable choisi parmi les groupements acrylate ou méthacrylate, un groupement époxy, un groupe isocyanate, hydroxy, vinyléther ($-O-CH=CH_2$) ou vinylester ($-CO-O-CH=CH_2$), et
  - ii) A1 et A2, identiques ou différents, représentent un groupement de formule $-CnH_2n-$, dans laquelle n est un nombre entier allant de 0 à 20 et un ou plusieurs groupes méthylène dudit groupement $-CnH_2n-$ pouvant être remplacés par un ou plusieurs atomes d'oxygène, et
  - iii) M1 désigne un groupement de formule générale (I') - $R_1$-$X_1$-$R_2$-$X_2$-$R_3$-$X_3$-$R_4$-, dans laquelle $R_1$, $R_2$, $R_3$ et $R_4$, identiques ou différents, désignent un groupement divalent choisi parmi -O-, -COO-, -CONH-, -CO-, -S-, -C≡C-, -CH=CH-, -N=N-, -N=N(O)-, et -$R_2$-$X_2$-$R_3$- ou -$R_2$-$X_2$- ou -$R_2$-$X_2$-$R_3$-$X_3$- pouvant également être une liaison covalente simple, et $X_1$,$X_2$,$X_3$ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B1 et/ou B2 et/ou B3, ledit hétéroarylène contenant de 1 à 3 hétéroatomes choisis parmi les atomes O, N et S, les groupements cycloalkylènes ayant de 3 à 10 atomes de carbones éventuellement substitués par -B1 et/ou -B2 et/ou -B3, -B1, -B2, et -B3 , identiques ou différents, étant choisis parmi les groupes alkyle en $C_1$-$C_{20}$, alkoxy en $C_1$-$C_{20}$, alkylthio en $C_1$-$C_{20}$, alkyl($C_1$-$C_{20}$) carbonyl; alkoxy($C_1$-$C_{20}$) carbonyl, alkyl($C_1$-$C_{20}$) thiocarbonyl, -OH, -F, -Cl, -Br, -I, -CN, $-NO_2$, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,

et

- b) au moins un deuxième monomère B chiral de formule (II) V1-A'1-W1-Z-W2-A'2-V2, dans laquelle

  - i) V1 et V2 , identiques ou différents, désignent un groupement choisi parmi un groupement acrylate ou méthacrylate, un groupement époxy, un groupement vinyléther ou vinylester, un groupement isocyanate, un alkyle en $C_1$-$C_{20}$, un alkoxy en $C_1$-$C_{20}$, un alkylthio en $C_1$-$C_{20}$, un alkoxy($C_1$-$C_{20}$) carbonyl, un alkyl($C_1$-$C_{20}$) thiocarbonyle, -OH, -F, -Cl, -Br, -I, -CN, -NO2, formyle, acétyle et des groupements alkyle, alkoxy ou alkylthio, ayant de 1 à 20 atomes de carbone, et interrompus par un ou plusieurs atome(s) d'oxygène ou un ou plusieurs atome(s) de soufre ou un ou plusieurs groupe(s) ester (-CO-O-),
  et au moins V1 ou V2 désigne un groupe polymérisable choisi parmi les groupements acrylate ou méthacrylate, un groupement époxy, un groupe isocyanate, hydroxy, vinyléther ($-O-CH=CH_2$) ou vinylester ($-CO-O-CH=CH_2$),
  - ii) A' 1 et A'2 , identiques ou différents, représentent un groupement de formule $-C_nH_{2n}-$, dans laquelle n est un nombre entier allant de 0 à 20 et un ou plusieurs groupes méthylène dudit groupement $C_nH_{2n}$ pouvant être remplacés par un ou plusieurs atomes d'oxygène, et
  - iii) W1 et W2 désignent un groupement divalent de formule générale $R'_1$-$X'_1$-$R'_2$-$X'_2$-$R'_3$- dans laquelle $R'_1$ , $R'_2$ et $R'_3$, identiques ou différents, désignent un groupement divalent choisi parmi -O-, -COO-, -CONH-, -CO-, -S-, -C≡C-, -CH=CH-, - N=N-, -N=N(O)-, et $R'_1$, $R'_2$ , $R'_3$ ou $R'_2$-$X'_2$ peut également être une liaison covalente simple, et $X'_1$ et $X'_2$ sont des groupements, identiques ou différents, choisis parmi les groupements 1,4-phénylène, 1,4-cyclohexylène, les groupements arylènes ou hétéroarylènes ayant un noyau aryle comprenant de 6 à 10 atomes éventuellement substitués par B'1 et/ou B'2 et/ou B'3, ledit hétéroarylène contenant de 1 à 3 hétéroatomes choisis parmi les atomes O, N et S, les groupements cycloalkylènes ayant de 3 à 10 atomes de carbones éventuellement substitués par -B'1 et/ou -B'2 et/ou -B'3, -B'1, -B'2, et -B'3, identiques ou différents,

étant choisis parmi les groupes alkyle en $C_1$-$C_{20}$, alkoxy en $C_1$-$C_{20}$, alkylthio en $C_1$-$C_{20}$, alkyl($C_1$-$C_{20}$)carbonyl, alkoxy ($C_1$-$C_{20}$)carbonyl, alkyl($C_1$-$C_{20}$)thiocarbonyl, -OH, -F, -Cl, -Br, -I, -CN, -$NO_2$, formyle, acétyle, et des groupements alkyle, alkoxy ou alkylthio ayant de 1 à 20 atomes de carbone, interrompus par un ou plusieurs atome(s) d'oxygène ou un ou plusieurs atome(s) de soufre ou un ou plusieurs groupe(s) ester,
et Z désigne un groupement chiral divalent comprenant au moins 4 atomes de carbone, notamment de 4 à 20 atomes de carbone, et mieux de 4 à 10 atomes de carbone, (le groupement chiral divalent comprenant au moins un carbone asymétrique, notamment un ou deux carbones asymétriques, et en particulier deux carbones asymétriques) et en particulier un groupe chiral divalent issu du groupe des dianhydrohexites, hexoses, pentoses, les dérivés binaphthyle (groupements binaphtyle), les dérivés biphényle (groupements biphényle), les dérivés d'acides tartriques ou des glycols optiquement actifs.

De préférence, le polymère à cristaux liquides est obtenu par polymérisation d'un mélange de monomères comprenant :

- a) au moins un premier monomère A de formule (I) Y1-A1-M1-A2-Y2
  dans laquelle

  - i) Y1 et Y2 , identiques ou différents, représentent un groupe acrylate ou méthacrylate, de préférence un groupe acrylate ;
  - ii) A1 et A2, identiques ou différents, représentent un groupement de formule -$C_nH_{2n}$-, dans laquelle n est un nombre entier allant de 1 à 20, de préférence allant de 2 à 6, et mieux égal à 4 ;
  - iii) M1 désigne un groupement de formule générale (I') - $R_1$-$X_1$-$R_2$-$X_2$-$R_3$-$X_3$-$R_4$-, dans laquelle R1 et R4 désignent -O-, $R_2$ et $R_3$ désignent -COO-,

  et X1,X2,X3 sont un groupement 1,4 phénylène, le groupe carbonyl -CO- respectivement de $R_2$ et de $R_3$ étant lié respectivement au groupement X1, X3 ,
  et
- b) au moins un deuxième monomère B chiral de formule (II) V1-W1-Z-W2-V2, dans laquelle

  - i) V1 désigne un groupement acrylate ou méthacrylate, et de préférence un groupe acrylate, et V2 désigne un groupement alkyle en $C_1$-$C_{20}$, un alkoxy en $C_1$-$C_{20}$, un alkoxy($C_1$-$C_{20}$)carbonyle, -OH, et de préférence désigne un groupement alkoxy en $C_1$-$C_{20}$, notamment en $C_1$-$C_4$, et en particulier un groupement méthoxy ;
  ii) W1 représente un groupement divalent de formule -X'1-CO-O-,
  W2 représente un groupement divalent de formule -O-CO-X'1-,
  dans lesquelles X'1 désigne un groupe 1,4-phénylène,
  et Z désigne un groupement chiral à deux liaisons, issu du groupement dianhydrohexite, en particulier un radical divalent de formule :

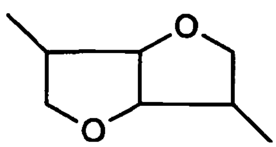

De préférence, le mélange de monomères comprend de 70 à 99 % en poids de monomère A et de 1 à 30 % en poids de monomère B, par rapport au poids total de monomère A et de monomère B, et mieux comprend de 90 à 95 % en poids de monomère A et de 5 à 10 % en poids de monomère B.

De préférence, la concentration des groupes polymérisables présents dans le mélange de monomère A et de monomère B (groupes polymérisables Y1, Y2 du monomère A et groupes polymérisables V1, V2 du monomère B) va de 3,2 à 15 mmoles/g.

Selon un mode particulier de réalisation de l'invention, le polymère à cristaux liquides est tel que le mélange de monomère A et de monomère B comprend des groupes polymérisables, dont au moins 90 % sont présents dans des monomères ayant au moins deux groupes polymérisables, en une concentration allant de 3,2 à 15 mmoles/g.

En particulier, le polymère à cristaux liquide comprend essentiellement ou consiste en un mélange des monomères A et B définis précédemment.

Le polymère à cristaux liquide présente notamment un pas d'hélice supérieur à 450 nm, notamment allant de 455 nm à 5000 nm, en particulier allant de 455 nm à 1000 nm, voire allant de 455 nm à 650 nm.

Le monomère A peut posséder un poids moléculaire moyen en poids allant de 150 à 800, et notamment allant de 460 à 625. Le monomère A peut être en particulier un dérivé de dibenzoate d'hydroquinone non substitué.

Le monomère B peut avoir un poids moléculaire moyen en poids allant de 500 à 1000, et notamment allant de 500 à 700.

Le polymère à cristaux liquides peut posséder un poids moléculaire moyen en poids inférieur à 625.

Le polymère à cristaux liquides défini précédemment peut être préparé suivant les procédés connus dans l'état de la technique, tels que ceux décrits dans les documents US 5362315, US 5807497, à partir du mélange de monomère décrit précédemment.

La polymérisation du mélange de monomères orienté peut, de manière déjà connue, se faire par exemple de façon radicalaire avec utilisation d'initiateurs thermiques du commerce, en utilisant des rayons d'électrons ou de la lumière UV en combinaison avec des photoinitiateurs du commerce, ou bien par des réactions d'addition ou de condensation.

La réticulation des mélanges de monomères, dans l'état structurel chiral, se fait de préférence au moyen d'une polyréaction qui, selon le type des groupes polymérisables, polycondensables ou polyadditionnables, se déroule sous la forme d'une polymérisation radicalaire, ionique ou catalysée au métal, ou d'une réaction de polycondensation ou d'une réaction de polyaddition.

L'initiation de la polymérisation radicalaire peut s'effectuer au moyen d'initiateurs correspondants ou par un rayonnement par UV, en utilisant des photoinitiateurs du commerce ou par un rayonnement à haute énergie, tel qu'un rayonnement d'électrons. Un avantage de la polymérisation thermique des radicaux ou de la polymérisation via un durcissement aux rayons d'électrons réside dans le fait qu'au mélange polymérisable peut également être ajouté un agent de protection contre la lumière, tel qu'un absorbeur d'UV (UVA) ou des capteurs de radicaux (HALS), pour stabiliser les pigments ou les films résultants face à la lumière UV, par exemple pour des applications extérieures, sans entraîner des pertes au niveau de la conversion de polymérisation, tel que ceci est le cas lors du durcissement aux UV, du fait de l'effet d'écrantage du photoinitiateur par un UVA. Il n'y a ainsi aucune diminution de la densité de réticulation.

Si le durcissement des films se fait de façon peroxydique ou par un rayonnement d'électrons, le mélange de monomères contient de préférence des agents de protection contre la lumière, du commerce, tels que des absorbeurs à UV ou des capteurs de radicaux, en une concentration globale de 0,5 à 5 % en poids.

Outre les photostabilisateurs, les mélanges de monomères peuvent également contenir d'autres additifs usuels visant à inhiber l'oxydation, à inhiber la polymérisation, ou des additifs visant à améliorer les propriétés rhéologiques. De plus, les charges absorbantes, telles que les pigments ou la suie, ainsi que des colorants ou des pigments à fluorescence peuvent être contenus.

Le film obtenu après la polymérisation est ensuite broyé en particules, notamment sous forme de plaquettes.

De préférence, les particules de polymère à cristaux liquides ont une plus grande taille allant de 1 μm à 3 mm, et de préférence allant de 30 μm à 500 μm. Ces particules sont avantageusement en forme de plaquettes.

Les particules peuvent être séparées (triées) par un procédé à sélectivité de la taille de grains.

De tels polymères et leurs particules sont décrits dans la demande EP-A-1046692.

Comme particules de polymère à cristaux liquides, on peut notamment utiliser celles connues sous le nom CTFA Polyacrylate-4 et vendus sous les dénominations « HELICONE® HC Sapphire », « HELICONE® HC Scarabeus », « HELICONE® HC Jade », « HELICONE® HC Maple », « HELICONE® HC XL Sapphire », « HELICONE® HC XL Scarabeus », « HELICONE® HC XL Jade », « HELICONE® HC XL Maple » par la société WACKER.

Les particules du polymère à cristaux liquide peuvent être présentes dans la composition selon l'invention à une teneur allant de 0,01 % à 99 % en poids, par rapport au poids total de la composition, notamment allant de 0,1 % à 60 % en poids, en particulier allant de 1 % à 30 % en poids, et voire allant de 5 % à 15 % en poids.

***Colorants monochromes liposolubles et hydrosolubles***

La composition peut comprendre, à titre de seconde matière colorante, au moins un colorant monochrome, notamment un colorant organique naturel tel que par exemple le carmin de cochenille, et/ou un colorant de synthèse tel que les colorants halogéno-acides, azoïques, anthraquinoniques. On peut également citer des colorants minéraux tels que le sulfate de cuivre ou de fer. On peut encore citer le brun Soudan, le rouge Soudan et le rocou, ainsi que le jus de betterave, le carotène et le bleu de méthylène.

Le colorant peut être présent dans la composition, seul ou en mélange, à raison de 0,001 à 15 % en poids, et notamment de 0,01 à 5 % en poids et notamment de 0,1 à 2 % en poids, par rapport au poids total de la composition.

***Les pigments***

Par pigments, il faut comprendre des particules blanches ou colorées, minérales ou organiques, destinées à colorer et/ou opacifier la composition.

Des pigments peuvent être présents dans la composition, à titre de seconde matière colorante, notamment à raison de 0,01 à 25 % en poids de la composition finale, et de préférence à raison de 3 à 10 % en poids.

Ils peuvent être blancs ou colorés, minéraux ou organiques, de taille usuelle ou nanométrique. Ils peuvent se présenter sous forme de poudre ou de pâte pigmentaire. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

***<u>Les nacres</u>***

Par nacres, il faut comprendre des particules irisées qui réfléchissent la lumière, notamment produites par certains mollusques dans leur coquille ou bien synthétisées.

Des nacres peuvent être présentes dans la composition, à titre de seconde matière colorante, notamment à raison de 0,01 à 20 % en poids, de préférence à un taux de l'ordre de 3 à 10 % en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

***<u>Les particules réfléchissantes</u>***

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de surbrillance visibles à l'oeil nu, c'est-à-dire des points plus lumineux qui contrastent avec leur environnement en semblant briller.

Des particules réfléchissantes peuvent être présentes dans la composition, à titre de seconde matière colorante, en étant dispersées de manière homogène par exemple à une teneur allant de 0,1 % à 20 % par rapport au poids total de la composition, notamment de 1 % à 15 % en poids, et en particulier de 1 % à 10 % en poids, par exemple environ 2 % notamment pour une composition destinée à être appliquée sur les lèvres.

Les particules réfléchissantes qui permettent une réflexion métallique de la lumière incidente conviennent tout particulièrement. C'est le cas notamment lorsque les particules réfléchissantes permettent quelque soit leur forme, une réflexion sur une couche d'un métal, par exemple de l'argent. De telles particules s'avèrent relativement neutres vis-à-vis de la couleur de la composition.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et en particulier d'au moins 80 %, voire 90 % ou encore 95 %.

La lumière réfléchie par les particules réfléchissantes peut être non iridescente, notamment dans le cas d'un reflet métallique.

Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant.

Le matériau réfléchissant peut comporter une couche de métal ou d'un composé métallique.

Des particules de verre recouvertes d'une couche métallique sont décrites notamment dans les documents JP-A-09188830, JP-A-10158450, JP-A-10158541, JP-A-07258460, JP-A-05017710.

La composition peut également comprendre, à titre de seconde matière colorante, au moins un composé photochrome mais distinct de ceux considérés selon l'invention c'est-à-dire caractérisé d'une manière générale par un $\Delta E < 5$ et le cas échéant une insolubilité dans la composition selon l'invention. On peut notamment citer les composés photochromes minéraux, et plus particulièrement les aluminosilicates dopés tels que la sodalite dopée par des halogènes, ou les oxydes ou hydrates métalliques tels que les oxydes de titane rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate. Un tel composé photochrome peut être incorporé dans la composition en une quantité de 0,001 à 20 % poids par rapport au poids total de la composition, de préférence en une quantité de 0,1 à 10 % en poids.

**MILIEU PHYSIOLOGIQUEMENT ACCEPTABLE**

Le milieu physiologiquement acceptable sera adapté à la nature de la surface à maquiller ou à traiter sur laquelle doit être appliquée la composition ainsi qu'à la forme sous laquelle la composition est destinée à être conditionnée, notamment solide ou fluide à température ambiante et pression atmosphérique.

La composition selon l'invention peut comprendre au moins une phase aqueuse et/ou au moins une phase grasse.

La composition selon l'invention peut être anhydre, ou peut également comprendre une phase aqueuse, qui peut comprendre de l'eau, une eau florale telle que de l'eau de bleuet et/ou une eau minérale telle que l'eau de VITTEL, l'eau de LUCAS ou l'eau de LA ROCHE POSAY.

Ladite phase aqueuse peut comprendre de 0,1 à 14 % en poids, par rapport au poids total de la phase aqueuse, d'un monoalcool en $C_2$-$C_6$ comme l'éthanol, le propanol, le butanol, l'isopropanol et l'isobutanol.

**Huiles**

Généralement, la composition selon l'invention comprend au moins une phase huileuse qui peut comprendre une ou plusieurs huiles cosmétiquement acceptables.

Par huile cosmétiquement acceptable selon l'invention, on entend tout corps gras liquide à 25°C, 1 atm, et ayant un poids moléculaire supérieur ou égal à 160, notamment compris entre 170 et 106, voire entre 200 et 5.105, compatible avec une application sur la peau, les muqueuses (lèvres) et/ou les phanères (ongles, cils, sourcils, cheveux).

De préférence, la phase huileuse est macroscopiquement homogène, c'est-à-dire homogène à l'oeil nu.

La phase huileuse peut comprendre une ou plusieurs huiles, qui peuvent être polaires ou apolaires, volatiles ou non volatiles, et notamment, hydrocarbonées.

On entend par huile polaire, une huile composée de composés chimiques comportant au moins un groupement polaire. Les groupements polaires sont bien connus de l'homme du métier : il peut s'agir notamment de groupement de type alcool, ester ou acide carboxylique.

En particulier, on peut définir les huiles polaires selon l'invention comme ayant un paramètre moyen de solubilité $\delta a$ selon l'espace de solubilité de Hansen, à 25°C, de : $\delta a \geq 5{,}0\ (J/cm^3)^{½}$.

Les huiles polaires comprennent les huiles plutôt polaires qui ont un paramètre moyen de solubilité à 25°C de : $5{,}0 \leq \delta a \leq 7{,}0\ (J/cm^3)^{½}$, et les huiles franchement polaires qui ont un paramètre moyen de solubilité à 25°C de : $\delta a > 7{,}0\ (J/cm^3)^{½}$.

De la même manière, les huiles apolaires au sens de l'invention ont un paramètre moyen de solubilité $\delta a$ selon l'espace de solubilité de Hansen, à 25 °C, de :

$$0 \leq \delta a < 5{,}0\ (J/cm^3)^{½}.$$

Les huiles apolaires au sens de l'invention comprennent les huiles franchement apolaires ($\delta a = 0$) et les huiles peu polaires qui ont un paramètre moyen de solubilité à 25°C de : $0 < \delta a < 5{,}0\ (J/cm^3)^{½}$.

Ainsi, plus la valeur de $\delta a$ est élevée, plus la polarité de l'huile est élevée.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta D$ caractérise les forces de dispersion de LONDON issues de la formation de dipôles induits lors des chocs moléculaires ;
- $\delta p$ caractérise les forces d'interactions de DEBYE entre dipôles permanents ainsi que les forces d'interactions de KEESOM entre dipôles induits et dipôles permanents;
- $\delta h$ caractérise les forces d'interactions spécifiques (type liaisons hydrogène, acide/base, donneur/accepteur, etc.) ;
- $\delta a$ est déterminé par l'équation : $\delta a = (\delta p^2 + \delta h^2)^{½}$

Les paramètres $\delta p$, $\delta h$, $\delta D$ et $\delta a$ sont exprimés en $(J/cm^3)^{½}$.

Lorsque la phase huileuse est un mélange de différentes huiles, les paramètres de solubilité du mélange sont déterminés à partir de ceux des composés pris séparément, selon les relations suivantes :

$$\delta_{Dmel} = \sum_i xi\, \delta Di \quad ; \quad \delta = \sum_i xi\, \delta pi \quad \text{et} \quad \delta = \sum_i xi\, \delta hi$$

$$\delta_{amel} = (\delta^2_{pmel} + \delta^2_{hmel})1/2$$

où xi représente la fraction volumique du composé i dans le mélange.

Il est à la portée de l'homme du métier de déterminer les quantités de chaque huile pour obtenir une phase huileuse satisfaisant aux critères souhaités.

Selon un premier mode de réalisation de l'invention, la phase huileuse est polaire et peut comprendre principalement, voire exclusivement, une ou plusieurs huiles polaires (plutôt ou franchement polaires) en mélange, qui peuvent donc représenter 5 à 100 % en poids, notamment de 10 à 90 %, voire de 15 à 60 % et en particulier de 20 à 50 % en poids du poids total de ladite phase huileuse.

Selon ce premier mode, la phase huileuse polaire présente un paramètre moyen de solubilité δa selon l'espace de solubilité de Hansen, à 25 °C, supérieur ou égal à 5,0 $(J/cm^3)$½, notamment supérieur ou égal à 5,3, voire supérieur ou égal à 5,5 et encore mieux supérieur ou égal à 6,0 $(J/cm^3)$½, voire supérieur ou égale à 7,0 $(J/cm^3)$½.

Selon un second mode de réalisation de l'invention, la phase huileuse est apolaire et peut comprendre de 5 à 100 % en poids, notamment de 10 à 90 %, voire de 15 à 60 % et en particulier de 20 à 50 % en poids d'une ou plusieurs huiles apolaires (apolaires ou peu polaires); elle présente un paramètre moyen de solubilité δa selon l'espace de solubilité de Hansen, à 25 °C, inférieur à 5,0, notamment inférieur ou égal à 4,9, encore mieux inférieur ou égal à 4,5 et encore mieux inférieur ou égal à 4,0 $(J/cm^3)$½.

Les huiles susceptibles d'être employées dans la phase huileuse peuvent être choisies parmi, seule ou en mélange, les huiles d'origine animale, végétale, minérale ou synthétique, volatiles ou non.

On entend par huile volatile, les huiles ayant, à 25 °C, une pression de vapeur comprise entre 0,02 et 300 mm Hg (soir 2,66 à 40000 Pa). De préférence, on utilise des huiles volatiles dont le point éclair est de l'ordre de 30-100°C.

On peut notamment citer :

- les huiles animales ou végétales notamment formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, de calophyllum, de macadamia, de colza, de coprah, d'arachide, de palme, de ricin, de jojoba, d'olive ou de germes de céréales; l'huile de beurre de karité; le perhydrosqualène;
- les esters et les éthers de synthèse notamment d'acides gras comme par exemple les huiles de formule $R_1COOR_2$ dans laquelle $R_1$ représente le reste d'un acide gras supérieur comportant de 7 à 29 atomes de carbone et $R_2$ représente une chaîne hydrocarbonée contenant de 3 à 30 atomes de carbone comme par exemple le myristate d'isopropyle, le palmitate d'éthyl-2-hexyle, le stéarate d'octyl-2-dodécyle, l'érucate d'octyl-2-dodécyle, l'isononanoate d'isononyle, l'isostéarate d'isostéaryle; les esters hydroxylés comme le lactate d'isostéaryle, l'hydroxystéarate d'octyle, l'hydroxystéarate d'octyldodécyle, le malate de diisostéaryle, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; les esters du pentaérythritol comme le tétraisostéarate de pentaérythrithyle ; des esters du type trimellitate de tridécyle ;
- des alcools gras notamment ayant de 12 à 26 atomes de carbone comme l'octyldodécanol, le 2-butyloctanol, le 2-hexyldécanol, le 2-undécylpentadécanol, l'alcool oléique ;
- des hydrocarbures linéaires ou ramifiés, d'origine minérale ou synthétique tels que les huiles de paraffine et leurs dérivés, la vaseline, les polydécènes, le polyisobutène hydrogéné tel que le parléam; les isoparaffines comme l'isohexadécane et l'isodécane ;
- des glycérides et notamment des acétylglycérides ou des triglycérides d'acides gras ayant 4 à 10 atomes de carbone, comme les triglycérides des acides heptanoïque, octanoïque et des acides caprique/caprylique,
- et leurs mélanges.

Parmi les huiles polaires particulièrement préférées, on peut citer l'octyldodécanol, l'hexyldécanol, l'octyldécanol, l'alcool oléique, l'huile de ricin, le malate de diisostéaryle, le triheptanoate de glycéryle, le trioctanoate de glycéryle, le triglycéride des acides caprique/caprylique, le triisononanoïn, le trimellitate de tridécyle, et leurs mélanges.

Parmi les huiles apolaires particulièrement préférées, on peut citer les hydrocarbures aliphatiques notamment en $C_6$-$C_{40}$ comme les huiles de paraffine, volatiles, telles que l'isohexadecane ou l'isododécane, ou non volatiles, et leurs dérivés; la vaseline, les polydécènes hydrogénés ou non, le polyisobutène hydrogéné tel que l'huile de Parléam, le squalane, les polybutylènes, l'isononanoate d'isononyle; les huiles fluorées notamment perfluorées, et leurs mélanges.

On peut notamment citer les huiles suivantes :

| | δa (J/cm$^3$)½ |
|---|---|
| huile de ricin | 9,09 |
| 2-hexyldécanol | 8,55 |
| alcool oléïque | 8,17 |
| octyldodécanol | 7,69 |
| Triglycéride de l'acide heptanoïque | 7,29 |
| diisostéarylmalate | 7,19 |
| Triglycéride de l'acide octanoïque | 6,87 |
| Triglycéride des acides caprique/caprylique | 6,69 |
| Triisononanoïne | 6,54 |
| Trimellilate de tridécyle | 5,35 |
| Isononanoate d'isononyle | 4,87 |
| Polyisobutène hydrogéné | 0 |
| Isododécane | 0 |

On choisit de préférence la phase huileuse de manière à ce que la ou les matières colorantes photochromes y soient à défaut de solubles, dispersibles en tant que telles c'est-à-dire sans traitement de surface annexe.

La phase huileuse est de préférence comprise en une quantité de 1 à 99 % en poids, notamment 10 à 90 % en poids, de préférence 15 à 80 % en poids, par rapport au poids total de la composition cosmétique.

La composition selon l'invention peut comprendre en outre d'autres corps gras que les huiles ci-dessus, qui peuvent être choisis par l'homme du métier sur base de ses connaissances générales, de manière à conférer à la composition finale les propriétés souhaitées, par exemple en consistance et/ou en texture.

Ces corps gras additionnels peuvent être des cires, des gommes et/ou des corps gras pâteux d'origine animale, végétale, minérale ou synthétique, ainsi que leurs mélanges.

Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, généralement une dureté supérieure à 0,5 MPa, et présentant à l'état solide une organisation cristalline anisotrope.

On peut notamment citer les cires d'origine animale, végétale, minérale ou synthétique telles que les cires microcristallines, la cire de paraffine, le pétrolatum, la vaseline, l'ozokérite, la cire de montan; la cire d'abeilles, la cire de lanoline et ses dérivés; les cires de Candellila, d'Ouricury, de Carnauba, du Japon, le beurre de cacao, les cires de fibres de lièges ou de canne à sucre, la cire de lignite, la cire de son de riz, la cire de sapin, la cire de coton; les huiles hydrogénées ayant une température de fusion supérieure à 40°C (environ), comme l'huile de jojoba hydrogénée; les esters gras et les glycérides concrets à 25°C; les cires de polyéthylène et les cires obtenues par synthèse de Fischer-Tropsch; des huiles hydrogénées concrètes à 25°C; des lanolines.

Les corps gras pâteux ont généralement un point de fusion compris entre 25 et 60°C, de préférence entre 30 et 45°C, et/ou une dureté allant de 0,001 et 0,5 MPa, de préférence entre 0,005 et 0,4 MPa. On peut notamment citer les lanolines et leurs dérivés, ou les esters de cholestérol.

Ces corps gras additionnels peuvent être présents en une quantité de 0,1 à 50 % en poids, notamment de 3 à 40 % en poids, encore mieux de 5 à 30 % en poids, par rapport au poids total de la composition.

**Tensioactifs**

La composition selon l'invention peut éventuellement comprendre un tensioactif, notamment lorsqu'elle se présente sous forme d'émulsion, en particulier en une quantité de 0,01 à 30 % en poids par rapport au poids total de la

composition.

On peut citer, seuls ou en mélange, les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, $\alpha$-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates. Le radical alcoyle ou acyle dans tous ces composés désigne généralement une chaîne de 12 à 18 atomes de carbone.

On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques.

On peut encore citer les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras (stéarate, oléate) du sorbitan oxyéthylénés ou non, des esters d'acides gras du saccharose, des esters d'acides gras de polyéthylèneglycol (monostéarate ou monolaurate de polyéthylène glycol); des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.

On peut également citer le trioléyl phosphate; les ester d'acides gras de polyols comme les mono-, di-, tri- ou sesqui-oléates ou stéarates de sorbitol ou de glycérol, les laurates de glycérol ou de polyéthylène glycol; les alkyl ou alkoxy diméthicones copolyols à chaîne alkyle ou alkoxy pendante ou en bout de squelette siliconé ayant par exemple de 6 à 22 atomes de carbone; les alkyl (lauryl, cétyl, stéaryl, octyl) éthers polyoxyéthylénés et les diméthicones copolyols.

**Agent épaississant**

La composition selon l'invention peut encore comprendre un ou plusieurs agents épaississants, par exemple dans des concentrations de 0,01 à 6 % en poids par rapport au poids total de la composition.

L'agent épaississant peut être choisi parmi, seul ou en mélange :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryle tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octyl-acrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium;
- les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium ;
- la silice éventuellement modifiée;
- les galactomannanes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-HANCE-AG ;
- les dérivés de cellulose tel que l'éthylcellulose.
- les copolymères séquencés, notamment de type "dibloc" ou "tribloc" du type polystyrène/polyisoprène, polystyrène/polybutadiène, polystyrène/copoly(éthylène-butylène) ou encore polystyrène/copoly(éthylène-propylène) tels que ceux vendus sous le nom de 'Kraton' par Shell Chemical;
- les polymères de type polyamide, par exemple comportant un squelette polymérique ayant des motifs répétitifs amide, et éventuellement au moins une chaîne grasse pendante et/ou au moins une chaîne terminale éventuellement fonctionnalisées, ayant de 8 à 120 atomes de carbone et étant liées à ces motifs amide, parmi lesquels on peut citer les produits commerciaux vendus par la société Arizona Chemical sous les noms Uniclear 80 et Uniclear 100

qui sont un mélange de copolymères d'un diacide en C36 condensé sur l'éthylène diamine, de masse moléculaire moyenne en poids d'environ 6000, les groupes ester terminaux résultant de l'estérification des terminaisons d'acide restantes par l'alcool cétylique, stéarylique ou leurs mélanges (appelés aussi alcool cétylstéarylique).

## Polymère filmogène

Selon l'application envisagée, la composition peut comprendre en outre au moins un polymère filmogène. Ceci est généralement le cas lorsque l'on souhaite préparer une composition de type vernis à ongles, mascara, eye-liner ou laque à lèvres.

Parmi les polymères filmogènes utilisables dans la composition de la présente invention, on peut citer les polymères synthétiques, de type radicalaire ou de type polycondensat, les polymères d'origine naturelle, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène. En particulier, ces monomères peuvent être polymérisés avec des monomères acides et/ou leurs esters et/ou leurs amides, tels que ceux mentionnés précédemment.

Parmi les polycondensats filmogènes, on peut citer les polyuréthanes, les polyesters, les polyesters amides, les polyamides, et les résines époxyesters, les polyurées.

Les polymères d'origine naturelle, éventuellement modifiés, peuvent être choisis parmi la résine shellac, la gomme de sandaraque, les dammars, les élémis, les copals, les polymères cellulosiques insolubles dans l'eau tels que la nitrocellulose, l'acétate de cellulose, l'acétobutyrate de cellulose, l'acétopropionate de cellulose, l'éthyl cellulose, et leurs mélanges.

Les polymères peuvent être dissous ou dispersés dans le milieu cosmétiquement acceptable. Les polymères peuvent être présents à une teneur allant de 0,01 % à 40 % en poids par rapport au poids total de la composition.

La composition peut également comprendre un plastifiant choisi parmi les plastifiants usuels et qui peut être présent à une teneur allant de 0,1 à 40 % en poids par rapport au poids total de la composition.

## Charge

La composition peut comprendre en outre des charges habituellement utilisées dans les compositions cosmétiques.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes à raison de 0,01 à 60 % en poids, de préférence de 3 à 10 %, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

## LES ADDITIFS

La composition peut en outre comprendre un filtre UV qui peut être incorporé dans la composition en une quantité de 0,01 à 20 % en poids par rapport au poids total de la composition, de préférence en une quantité de 0,1 à 10 % en poids. On peut notamment citer, parmi les filtres solaires susceptibles d'être employés, les composés appartenant aux familles des para-aminobenzoïques; des salicylates; des dibenzoylméthanes; des cinnamates; des dérivés de β, β'-diphénylacrylate; des benzophénones; des benzylidène camphres; des phényl benzimidazoles; des triazines; des phényl benzotriazoles; des anthraniliques; des imidazolines et/ou des benzalmalonates.

La composition selon l'invention peut également être exempte de filtre U.V.

La composition peut comprendre en outre tout additif usuellement utilisé dans le domaine cosmétique, tel que

des antioxydants, des parfums, des huiles essentielles, des conservateurs, des actifs cosmétiques lipophiles ou hydrophiles, des hydratants, des vitamines, des sphingolipides, des agents autobronzants tels que la DHA, des azurants optiques, des agents antimousses, des agents séquestrants.

Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

## FORMULATION GALENIQUE

La composition cosmétique selon l'invention trouve notamment une application particulièrement intéressante dans le domaine du maquillage de la peau, des semi-muqueuses, des muqueuses et/ou des phanères (ongles, cils, sourcils, poils et cheveux).

Elle peut comprendre ou se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.

Elle trouve une application particulière dans le domaine des rouges à lèvres, des fonds de teint, des fards à joues ou à paupières, des poudres libres ou compactes, des crèmes teintées, des produits de maquillage du corps, des produits de coloration de la peau, des eye-liners et des mascaras.

La composition cosmétique selon l'invention peut être obtenue selon les procédés de préparation classiquement utilisés en cosmétique.

Les exemples soumis ci-après sont présentés à titre illustratif et non limitatif du domaine de l'invention.

## EXEMPLES

Les pourcentages ci-après sont tous exprimés en poids par rapport au poids total de la composition.

Les matières colorantes photochromes utilisées dans les formulations ci-après sont des dérivés naphtopyranes de la société James Robinson commercialisés sous les dénominations suivantes :

| | |
|---|---|
| Reversacol Corn Yellow | Réf. 1 |
| Reversacol Flame | Réf. 2 |
| Reversacol Aqua Green | Réf. 3 |
| Reversacol Ruby | Réf. 4 |

Leurs ΔE respectifs ont été appréciés selon le protocole décrit dans la description. Le tableau I rend compte des écarts de teinte (ΔE) obtenus après 2 minutes d'exposition.

| Photochromes | ΔE |
|---|---|
| Réf. 1 | 51 |
| Réf. 2 | 53 |
| Réf. 3 | 6 |
| Réf . 4 | 49 |

Pour vérifier les propriétés effet de couleur dynamique d'une composition conforme à l'invention, on a réalisé une formulation de fond de teint ayant la composition suivante :

*Fond de teint*

| Composition | % |
|---|---|
| Eau | Qsp 100 |
| Veegum | 0,7 |
| Propylène glycol | 6 |
| Polysorbate 20 | 3,7 |
| Oxydes de fer | 0,4 |
| Colorants photochromes : Réf 1, 2 et 3 | 0,1 |
| Isononanoate d'isononyle | 22 |
| Acide stéarique | 1 |
| Méthyl glucose sesquistearate | 3,5 |
| Silicone | 10 |

Les trois colorants photochromes y ont été simultanément introduits ; Réf 1 à raison de 0,07 % en poids, Réf 2 à raison de 0,02 % en poids et Réf 3 à raison de 0,01 %.

L'efficacité en terme de dynamique de couleur est vérifiée pour la composition photochrome correspondante. Il suffit donc d'incorporer une faible concentration notamment de l'ordre de 0,1 % en poids de(s) colorant(s) photochrome (s) par rapport au poids total de la composition pour que la crème faiblement teintée à l'origine, se fonce en quelques secondes sous exposition au soleil.

*Rouge à lèvres en bâtonnet*

| | % en poids |
|---|---|
| Néopentanoate d'octyldodécyle | 17,0 |
| Triglycéride d'acide caprique/caprylique | 10,2 |
| Huile de lanoline | 15,0 |
| Lanoline acétylée | 10,2 |
| Polybutène | 15,0 |
| Colorant photochrome (Réf. 4) | 2,0 |
| Particules de verre enrobées d'argent (METASHINE®)* | 2,0 |
| Pigment goniochromatique (SICOPEARL®)** | 3,0 |
| Cire microcristalline | 2,5 |
| Cire de polyéthylène | 7,4 |
| Phényl triméthicone | 7,0 |
| Polyisobutène hydrogéné | 6,5 |
| Parfum, conservateur, antioxydant | qs |

* commercialisé par la société TOYAL

** commercialisé par la société BASF

On obtient un bâtonnet de rouge à lèvres présentant également de bonnes propriétés dynamiques de couleur en réponse à une excitation lumineuse UV.

## Revendications

1. Composition cosmétique comprenant dans un milieu physiologiquement acceptable au moins deux matières colorantes, dont au moins une matière colorante photochrome **caractérisée en ce que** ladite matière colorante photochrome présente un écart de teinte $\Delta E$ au moins égal à 5 et est au moins un dérivé naphtopyrane présent dans la composition sous une forme solubilisée et de type 2H-naphto-[2,1-b]-pyrane de formule (I) ou 3H-naphto-[2,1-b]-pyrane de formule (II) :

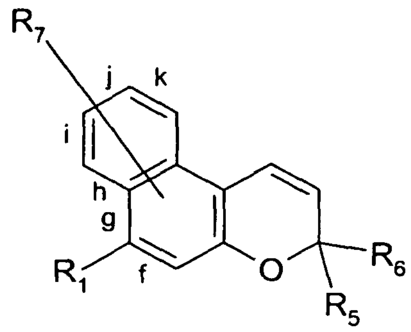

(I)

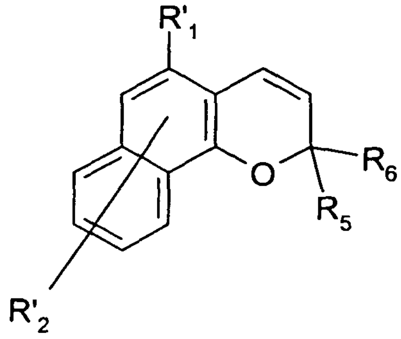

(II)

dans lesquelles :

- R1 représente :

  - (i) un atome d'hydrogène;
  - (ii) un groupement hydrocarboné ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné;
  - (iii) un cycle hydrocarboné formé avec l'une des liaisons "f" ou "gh" et le radical $R_7$; ou
  - (iv) un groupement choisi parmi $-COOR_4$, $-C(O)NR_2R_3$, $-NR_2R_3$, $-OR_4$ et - $SR_4$, dans lequel :

    - $R_2$ et $R_3$ soit représentent, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,

soit pris ensemble avec l'atome d'azote auquel ils sont reliés, forment un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20 atomes de carbone, linéaire, ramifié ou cyclique, saturé ou insaturé, comportant éventuellement 1 à 5 hétéroatomes choisis parmi N, O, S, Si et P;
- $R_4$ représente un groupement hydrocarboné ayant 1 à 20 atomes de carbone, linéaire, ramifié ou cyclique, saturé ou insaturé, éventuellement halogéné ou perhalogéné, et/ou éventuellement comportant 1 à 5 hétéroatomes choisis parmi N, O, S, Si et P;
- $R_5$ et $R_6$ représentent, indépendamment l'un de l'autre, un groupement choisi parmi :

  - (i) les groupements aminoaryles cycliques saturés de formule (IIA) ou (IIB) :

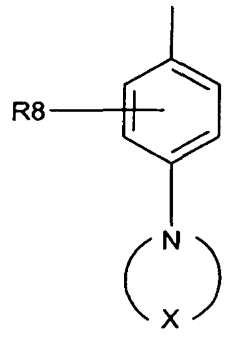

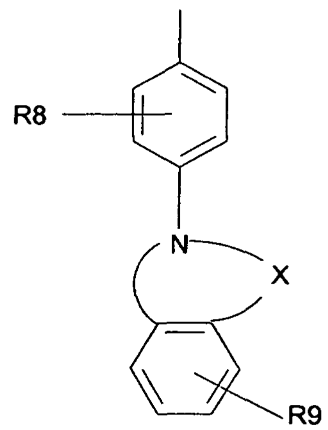

(IIA) (IIB)

dans lesquelles le cycle comportant N et X est un cycle saturé qui comprend au total 3 à 30 atomes inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;

- (ii) les groupements indolinoaryles de formule (III) :

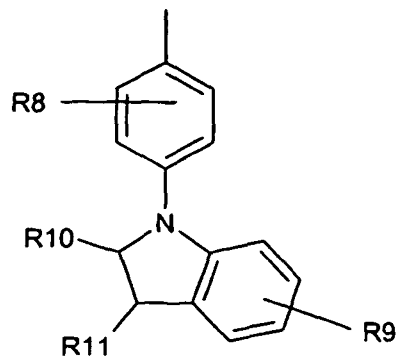

(III)

dans laquelle $R_{10}$ et $R_{11}$ représentent, indépendamment l'un de l'autre, un groupement choisi parmi (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), $-NO_2$ (nitro); (iv) un atome d'hydrogène; (v) un groupement choisi parmi - $C(O)NR_2R_3$, $-NR_2R_3$, $-OR_4$ ou $-SR_4$ avec $R_2$, $R_3$ et $R_4$ ayant les significations données ci-dessus; (vi) les radicaux $R_{10}$ et $R_{11}$ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

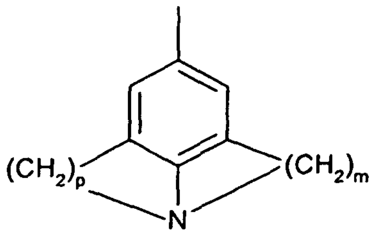

(IV)

dans laquelle m et p sont, indépendamment l'un de l'autre, des entiers allant de 2 à 5;

- (iv) les groupements aminoaryles cycliques insaturés de formules (VA), (VB) ou (VC) :

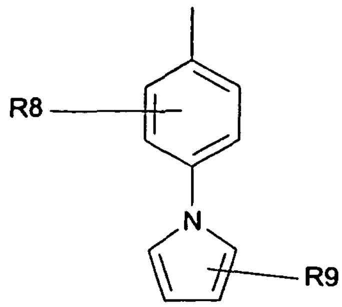

(VA)

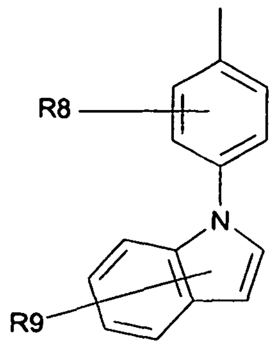

(VB)

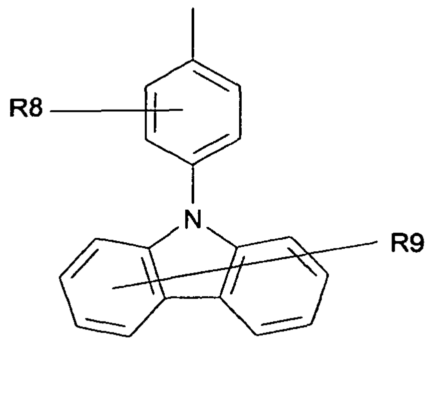

(VC)

dans lesquelles $R_8$ et $R_9$, représentent, indépendamment l'un de l'autre, un groupement choisi parmi (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), $-NO_2$ (nitro); (iv) un atome d'hydrogène; (v) un groupement choisi parmi - $C(O)NR_2R_3$, $-NR_2R_3$, $-OR_4$ ou $-SR_4$ avec $R_2$, $R_3$ et $R_4$ ayant les significations données ci-dessus;

- (v) un groupement hydrocarboné ayant 1 à 30 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

- $R_7$ représente un groupement choisi parmi :

  - (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
  - (ii) les atomes d'halogène ;
  - (iii) les groupements -CN (nitrile), -COOH (carboxylate), $-NO_2$ (nitro); - N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus;
  - (vi) le radical $R_7$ pouvant en outre former, avec l'une des liaisons "i", "j", "k", ou "g,h" prises avec le radical $R_1$, ou "f" prise avec le radical $R_1$, un cycle hydrocarboné saturé ayant au total 3 à 8 atomes de carbone, éventuellement comportant 1 à 5 hétéroatomes choisis parmi N, O, S, Si et P;

- $R'_1$ représente un groupement choisi parmi :

  - (i) un atome d'hydrogène;
  - (ii) un groupement hydrocarboné ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
  - (iii) un groupement choisi parmi $-C(O)NR_2R_3$, $-NR_2R_3$, $-OR_4$ et $-SR_4$, avec $R_2$, $R_3$ et $R_4$ ayant les significations données ci-dessus;

- $R'_2$ représente un groupement choisi parmi :

  - (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
  - (ii) les atomes d'halogène ;
  - (iii) les groupements -CN (nitrile), -COOH (carboxylate), $-NO_2$ (nitro); - N=N- (azo); =NH (imino); $-CONH_2$ (amide);
  - (iv) un atome d'hydrogène;
  - (v) un groupement choisi parmi $-C(O)NR_2R_3$, $-NR_2R_3$, $-OR_4$ ou $-SR_4$ avec $R_2$, $R_3$ et $R_4$ ayant les significations données ci-dessus ;

la composition comprenant de 0,001 à 20 % en poids de ladite matière colorante photochrome, par rapport au poids total de la composition et
ledit paramètre ΔE étant déterminé selon le protocole et à l'aide de la composition témoin décrits dans la description et calculé selon :

$$\Delta E = [(L - L_o)^2 + (a - a_o)^2 + (b - b_o)^2]^{1/2}$$

dans laquelle ($L_0$, $a_0$ et $b_0$) figurent les coordonnées trichromatiques initiales de la composition témoin avant irradiation et (L, a et b) les coordonnées obtenues après arrêt de l'irradiation.

**2.** Composition selon la revendication 1, **caractérisée en ce que** ladite matière colorante photochrome est présente en une quantité efficace pour conférer un effet dynamique en terme de couleur et d'éclat à ladite composition.

**3.** Composition selon la revendication 1 ou 2, **caractérisée en ce** ledit dérivé naphtopyrane répond à la formule (Ia) ou (IIa) suivantes :

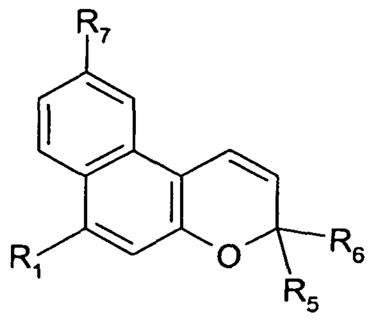

(Ia)

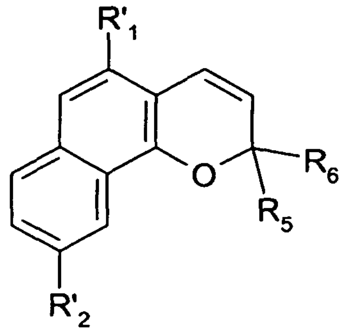

(IIa)

dans lesquelles $R_1$, $R_5$, $R_6$, $R_7$, $R'_1$ et $R'_2$ sont tels que définis tels en revendication 1.

**4.** Composition selon l'une quelconque des revendications précédentes, **caractérisée en ce que** $R_1$ représente un atome d'hydrogène; un cycle hydrocarboné avec l'une des liaisons "f" ou "gh" et le radical $R_7$; ou un groupement choisi parmi $-COOR_4$, $-NR_2R_3$, $-OR_4$ et $-SR_4$, dans lequel :

- $R_2$ et $R_3$ soit peuvent représenter, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,
soit pris ensemble avec l'atome d'azote auquel ils sont reliés, peuvent former un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20, atomes de carbone, linéaire, ramifié ou cyclique, saturé ou insaturé, comportant éventuellement 1 à 5 hétéroatomes choisis parmi N, O, S, Si et P;
- $R_4$ peut représenter un groupement hydrocarboné ayant 1 à 20 atomes de carbone, linéaire, ramifié ou cyclique, saturé ou insaturé, éventuellement halogéné ou perhalogéné et/ou éventuellement comportant 1 à 5 hétéroatomes choisis parmi N, O, S, Si et P.
et/ou
- $R_5$ et $R_6$ peuvent représenter, indépendamment l'un de l'autre, un groupement choisi parmi :

  - les groupements aminoaryles cycliques saturés de formule (IIA) ou (IIB) :

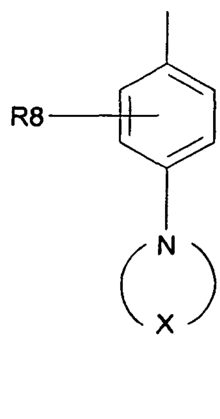

(IIA)

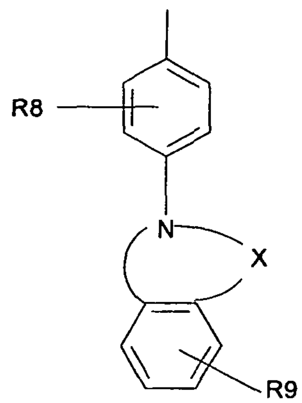

(IIB)

dans lesquelles le cycle comportant N et X est un cycle saturé qui comprend au total 3 à 30 atomes inclus

l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;

- un groupement hydrocarboné ayant 1 à 30 atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4-CONR_2R_3$, $-C_6H_4-NR_2R_3$ et $-C_6H_4-OR_4$ avec $R_2$, $R_3$ et $R_4$ ayant les significations données ci-dessus;

**5.** Composition selon l'une quelconque des revendications précédentes, **caractérisée en ce que** ladite matière colorante photochrome répond à la formule (I) ou (Ia) pour lesquelles :

- $R_1$ représente l'hydrogène; ou un groupement -COOR avec R étant un radical hydrocarboné saturé ayant 1 à 12 atomes de carbone ; ou un groupement

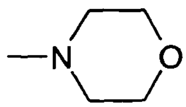

et/ou
- $R_5$ et $R_6$ représentent, indépendamment l'un de l'autre, soit (i) un groupement de formule (IIA) :

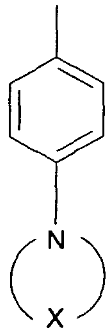

(IIA)

dans laquelle le cycle comportant N et X est un cycle saturé qui comprend au total 4 à 7 atomes inclus l'azote, et notamment 3 à 5 atomes de carbone et 0 ou 1 atome d'oxygène; et en particulier un groupement de formule:

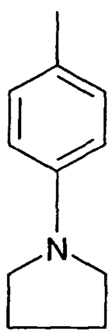

ou

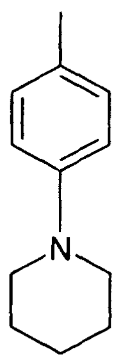

ou

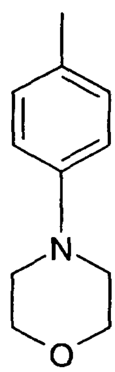

soit (ii) un groupement hydrocarboné ayant 5 à 14, notamment 6 à 10 atomes de carbone, linéaire, ramifié ou cyclique, saturé ou insaturé, éventuellement comportant 1 à 2 hétéroatomes choisis parmi N, O ou S;
et/ou

- $R_7$ représente un atome d'hydrogène ou un groupement $-NR_2R_3$, avec $R_2$ et $R_3$ représentant, indépendamment l'un de l'autre, un groupement hydrocarboné saturé, linéaire ou ramifié, ayant 1 à 12 atomes de carbone.

**6.** Composition selon l'une quelconque des revendications précédentes, **caractérisée en ce que** ladite matière colorante photochrome répond à la formule (II) ou (IIa) pour lesquelles :

- $R'_1$ représente l'hydrogène ou un groupement -COOR avec R étant un radical hydrocarboné saturé ayant 1 à 12 atomes de carbone ;
et/ou
- $R_5$ et $R_6$ représentent, indépendamment l'un de l'autre, soit (i) un groupement de formule (IIA) :

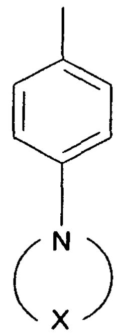
(IIA)

dans laquelle le cycle comportant N et X est un cycle saturé qui comprend au total 4 à 7 atomes inclus l'azote, soit (ii) un groupement hydrocarboné ayant 5 à 14 atomes de carbone, linéaire, ramifié ou cyclique, saturé ou insaturé, éventuellement comportant 1 à 2 hétéroatomes choisis parmi N, O ou S;
et/ou
- $R'_2$ représente l'hydrogène, ou un groupement -NR'R" avec R' et R", identiques ou différents, représentant un groupement hydrocarboné saturé, linéaire ou ramifié, ayant 1 à 12 atomes de carbone.

**7.** Composition selon l'une quelconque des revendications précédentes, **caractérisée en ce que** ladite matière colorante photochrome est choisie parmi

- le 3,3-di(4-méthoxyphényl)-6-morpholino-3H-naphto[2,1-b]pyrane
- le 3-phényl-3-(4-morpholinophényl)-6-morpholino-3H-naphto[2,1-b]pyrane
- le 3-phényl-3-(4-pipéridinophényl)-6-morpholino-3H-naphto[2,1-b]pyrane
- le 3-phényl-3-(4-pipéridinophényl)-6-carboxyméthyl-9-N-diméthyl-3H-naphto[2,1-b]pyrane
- le 2-phényl-2-(4-piperidinophényl)-5-carboxyméthyl-9-N-diméthyl-2H-naphto[1,2-b]pyrane et leurs mélanges.

**8.** Composition selon l'une quelconque des revendications précédentes, **caractérisée en ce que** ladite matière colorante photochrome comprend au moins deux dérivés naphtopyranes distincts.

**9.** Composition selon l'une quelconque des revendications précédentes, **caractérisée en ce qu**'elle associe à ladite matière colorante photochrome au moins une seconde matière colorante.

**10.** Composition selon la revendication précédente, **caractérisée en ce que** la seconde matière colorante est choisie parmi les agents de coloration goniochromatiques, colorants monochromes hydrosolubles ou liposolubles, pigments, particules réfléchissantes, nacres et leurs mélanges.

**11.** Composition selon l'une quelconque des revendications précédentes, **caractérisée en ce qu**'elle comprend, à titre de seconde matière colorante, au moins un agent de coloration goniochromatique.

**12.** Composition selon la revendication précédente, **caractérisée en ce qu'**elle comprend, de 0,1 à 60 % en poids dudit agent de coloration goniochromatique par rapport au poids total de la composition.

**13.** Composition selon la revendication 11 ou 12, **caractérisée en ce que** l'agent de coloration goniochromatique est choisi parmi les structures multicouches interférentielles et les agents de coloration à cristaux liquides.

**14.** Composition selon la revendication précédente, **caractérisée en ce que** l'agent de coloration goniochromatique à structures multicouche interférentielles comporte au moins deux couches, chaque couche, indépendamment ou non de la (ou les) autre(s) couche(s), étant réalisée à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

**15.** Composition selon la revendication 13, **caractérisée en ce que** l'agent de coloration à cristaux liquides est obtenu par polymérisation d'un mélange de monomères comprenant :

- a) au moins un premier monomère A de formule (I) Y1-A1-M1-A2-Y2
dans laquelle

  - i) Y1 et Y2 , identiques ou différents, représentent un groupe acrylate ou méthacrylate ;
  - ii) A1 et A2, identiques ou différents, représentent un groupement de formule $-CnH_2n-$, dans laquelle n est un nombre entier allant de 1 à 20 ;
  - iii) M1 désigne un groupement de formule générale (I') $-R_1-X_1-R_2-X_2-R_3-X_3-R_4-$, dans laquelle R1 et R4 désignent -O-, $R_2$ et $R_3$ désignent -COO-,

et X1,X2,X3 sont un groupement 1,4 phénylène, le groupe carbonyle -CO-respectivement de $R_2$ et de $R_3$ étant lié respectivement au groupement X1, X3,
et
- b) au moins un deuxième monomère B chiral de formule (II) V1-W1-Z-W2-V2, dans laquelle

  - i) V1 désigne un groupement acrylate ou méthacrylate, et V2 désigne un groupement alkyle en $C_1$-$C_{20}$, un alkoxy en $C_1$-$C_{20}$, un alkoxy($C_1$-$C_{20}$)carbonyle, -OH, ;
  ii) W1 représente un groupement divalent de formule -X' 1-CO-O-,

W2 représente un groupement divalent de formule -O-CO-X' 1-,
dans lesquelles X'1 désigne un groupe 1,4-phénylène,
et Z désigne un groupement chiral à deux liaisons, issu du groupement dianhydrohexite, en particulier un radical divalent de formule :

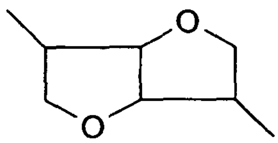

**16.** Composition selon la revendication précédente, **caractérisée en ce que** l'agent de coloration à cristaux liquides est obtenu à partir d'un mélange de monomères comprenant de 70 à 99 % en poids de monomère A et de 1 à 30 % en poids de monomère B, par rapport au poids total de monomère A et de monomère B.

**17.** Composition selon la revendication 13, 15 ou 16, **caractérisée en ce que** l'agent de coloration à cristaux liquides est présent à une teneur allant de 0,01 % à 99 % en poids par rapport au poids total de la composition.

**18.** Composition selon l'une quelconque des revendications précédentes, **caractérisée en ce qu**'elle comprend, à titre de seconde matière colorante, de 0,001 à 15% en poids d'au moins un colorant liposoluble ou hydrosoluble par rapport au poids total de la composition.

**19.** Composition selon l'une quelconque des revendications précédentes, **caractérisée en ce qu**'elle comprend, à titre de seconde matière colorante, de 0,01 à 25 % en poids d'au moins un pigment par rapport au poids total de la composition.

**20.** Composition selon l'une quelconque des revendications précédentes, **caractérisée en ce qu**'elle comprend, à titre de seconde matière colorante, de 0,01 à 20 % en poids de nacres par rapport au poids total de la composition.

**21.** Composition selon l'une quelconque des revendications précédentes, **caractérisée en ce qu**'elle comprend, à titre de seconde matière colorante, de 0,1 % à 20 % de particules réfléchissantes par rapport au poids total de la composition.

**22.** Composition selon l'une quelconque des revendications précédentes, **caractérisée en ce qu**'elle comprend au moins une phase huileuse.

**23.** Composition selon la revendication précédente, **caractérisée en ce que** la phase huileuse comprend une ou plusieurs huiles, polaires ou apolaires, volatiles ou non volatiles.

**24.** Composition selon la revendication 22 ou 23, **caractérisée en ce que** la phase huileuse comprend 5 à 100 % en poids d'huiles polaires par rapport du poids total de ladite phase huileuse.

**25.** Composition selon la revendication 22, 23 ou 24, **caractérisée en ce que** la phase huileuse comprend de 5 à 100 % en poids d'huiles apolaires par rapport du poids total de ladite phase huileuse.

**26.** Composition selon l'une quelconque des revendications 22 à 25, **caractérisée en ce que** les huiles peuvent être choisies parmi les huiles d'origine animale, végétale, minérale ou synthétique, volatiles ou non et leurs mélanges.

**27.** Composition selon la revendication précédente, **caractérisée en ce qu'** elles sont choisies parmi les huiles animales ou végétales, les esters et les éthers de synthèse notamment d'acides gras, les alcools gras, les hydrocarbures linéaires ou ramifiés, d'origine minérale ou synthétique, les glycérides et leurs mélanges.

**28.** Composition selon l'une quelconque des revendications 22 à 27, **caractérisée en ce que** la phase huileuse est telle que la ou les matières colorantes photochromes y sont solubles ou dispersibles.

**29.** Composition selon l'une quelconque des revendications 22 à 28, **caractérisée en ce qu**'elle comprend de 1 à 99 % en poids de phase huileuse par rapport au poids total de ladite composition.

**30.** Composition selon l'une quelconque des revendications précédentes, **caractérisée en ce qu**'elle comprend de 0,1 à 50 % en poids d'un corps gras autre qu'une huile par rapport au poids total de ladite composition.

**31.** Composition selon l'une quelconque des revendications précédentes, **caractérisée en ce qu**'elle est anhydre.

**32.** Composition selon l'une quelconque des revendications 1 à 30, **caractérisée en ce qu**'elle comprend au moins une phase aqueuse.

**33.** Composition selon la revendication précédente, **caractérisée en ce que** ladite phase aqueuse comprend de 0,1 à 14 % en poids, d'un monoalcool en $C_2$-$C_6$ par rapport au poids total de ladite phase aqueuse.

**34.** Composition selon l'une quelconque des revendications précédentes, **caractérisée en ce qu**'elle comprend au moins un tensioactif.

**35.** Composition selon la revendication précédente, **caractérisée en ce que** ledit tensioactif est présent en une quantité de 0,01 à 30 % en poids par rapport au poids total de la composition.

**36.** Composition selon l'une quelconque des revendications précédentes, **caractérisée en ce qu**'elle comprend au moins un agent épaississant.

**37.** Composition selon la revendication précédente, **caractérisée en ce que** ledit agent épaississant est présent en une quantité de 0,01 à 6 % en poids, par rapport au poids total de ladite composition.

**38.** Composition selon l'une quelconque des revendications précédentes, **caractérisée en ce qu**'elle comprend au moins un polymère filmogène.

**39.** Composition selon la revendication précédente, **caractérisée en ce que** ledit polymère filmogène est présent en une quantité de 0,01 % à 4 % en poids par rapport au poids total de la composition.

**40.** Composition selon l'une quelconque des revendications précédentes, **caractérisée en ce qu**'elle comprend au moins une charge.

**41.** Composition selon la revendication précédente, **caractérisée en ce que** ladite charge est présente à raison de 0,01 à 60 % en poids par rapport au poids total de ladite composition.

**42.** Procédé de maquillage de la peau, des lèvres et/ou des phanères comprenant l'application d'au moins une composition selon l'une quelconque des revendications précédentes sur la peau, les lèvres et/ou les phanères.

**43.** Utilisation d'au moins une matière colorante photochrome présentant un écart de teinte ΔE au moins égal à 5 et étant un dérivé naphtopyrane de formule (I) ou (II) tels que définis selon l'une quelconque des revendications 1 à 8 dans une composition cosmétique, notamment pour le maquillage de la peau, des phanères, des ongles et des lèvres contenant au moins une seconde matière colorante, pour modifier d'une manière réversible sa couleur en réponse à un rayonnement contenant des rayons ultraviolets, la composition comprenant de 0,001 à 20 % en poids de ladite matière colorante photochrome, par rapport au poids total de la composition.

## Claims

**1.** Cosmetic composition comprising, in a physiologically acceptable medium, at least two dyes, including at least one photochromic dye, **characterized in that** the said photochromic dye has a difference in hue ΔE at least equal to 5 and is at least one naphthopyran derivative being present in the said composition in a dissolved form and of 2H-naphtho[2,1-b]pyran type of formula (I) or 3H-naphtho[2,1-b]pyran type of formula (II):

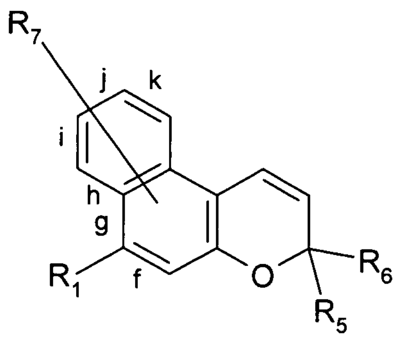

(I)

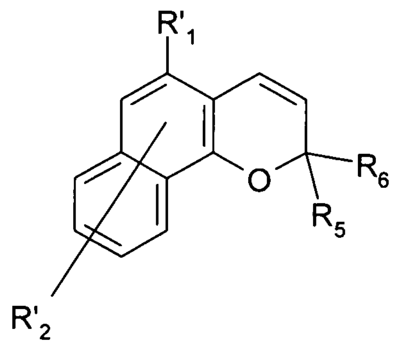

(II)

in which:

- $R_1$ represents:
- (i) a hydrogen atom;
- (ii) a linear, branched or cyclic, saturated or unsaturated hydrocarbon-based group containing from 1 to 30 carbon atoms, optionally comprising 1 to 5 hetero atoms chosen from N, O, S, Si and P, and/or optionally halogenated or perhalogenated;
- (iii) a hydrocarbon-based ring formed with one of the bonds "f" or "gh" and the radical $R_7$; or
- (iv) a group chosen from $-COOR_4$, $-C(O)NR_2R_3$, $-NR_2R_3$, $-OR_4$ and $-SR_4$, in which:
- $R_2$ and $R_3$ either represent, independently of each other, a linear, branched or cyclic, saturated or unsaturated hydrocarbon-based group containing 1 to 20 carbon atoms, optionally comprising 1 to 5 hetero atoms chosen from N, O, S, Si and P,
or, taken together with the nitrogen atom to which they are attached, form a saturated or unsaturated hydrocarbon-based heterocycle containing 3 to 10 carbon atoms and optionally 1 to 5 other hetero atoms chosen from N, O, S, Si and P, the said ring optionally being substituted with at least one linear, branched or cyclic, saturated or unsaturated hydrocarbon-based radical containing 1 to 20 carbon atoms, optionally comprising 1

to 5 hetero atoms chosen from N, O, S, Si and P;
- $R_4$ represents a linear, branched or cyclic, saturated or unsaturated hydrocarbon-based group containing 1 to 20 carbon atoms, which is optionally halogenated or perhalogenated , and/or optionally comprising 1 to 5 hetero atoms chosen from N, O, S, Si and P;
- $R_5$ and $R_6$ represent, independently of each other, a group chosen from:
- (i) the saturated cyclic aminoaryl groups of formula (IIA) or (IIB):

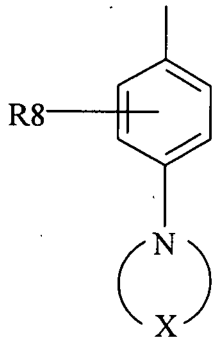

(IIA)

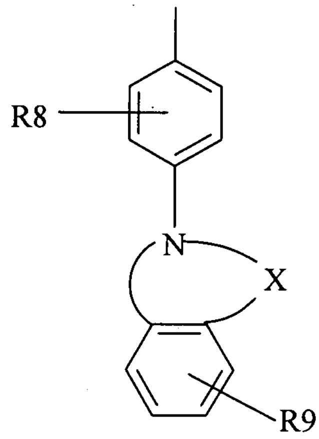

(IIB)

in which the ring comprising N and X is a saturated ring containing in total 3 to 30 atoms,, including the nitrogen, the remainder being carbon atoms and/or hetero atoms chosen from O, S, Si and P and/or groups chosen from -NH and -NR with R representing a linear, branched or cyclic, saturated or unsaturated hydrocarbon-based radical containing 1 to 20 carbon atoms, optionally comprising 1 to 5 hetero atoms chosen from N, O, S, Si and P;
- (ii) the indolinoaryl groups of formula (III):

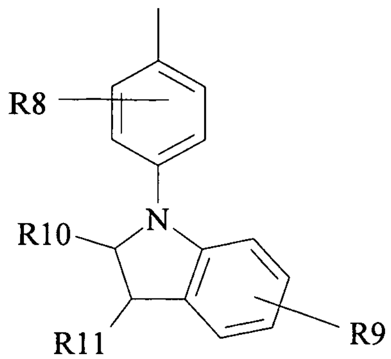

(III)

in which $R_{10}$ and $R_{11}$ represent, independently of each other, a group chosen from (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated; (ii) halogen atoms; (iii) - CN (nitrile), -COOH (carboxylate) or $NO_2$ (nitro) groups; (iv) a hydrogen atom; (v) a group chosen from -C(O)$NR_2R_3$, -$NR_2R_3$, -$OR_4$ and -$SR_4$ with $R_2$, $R_3$ and $R_4$ having the meanings given above; (vi) the radicals $R_{10}$ and $R_{11}$ together possibly forming a saturated or unsaturated hydrocarbon-based ring containing in total 5 to 8 atoms (including the atoms of the indoline ring), the said atoms being chosen from C, O, S and/or NR with R representing H or a linear, branched or cyclic, saturated or unsaturated hydrocarbon-based radical containing 1 to 20 carbon atoms, optionally comprising 1 to 5 hetero atoms chosen from N, O, S, Si and P,

- (iii) the groups of formula (IV):

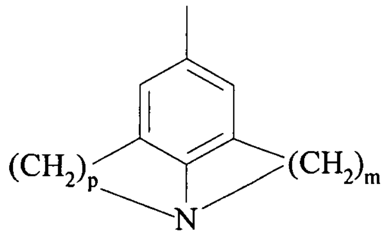

(IV)

in which m and p are, independently of each other, integers ranging from 2 to 5;

- (iv) the unsaturated cyclic aminoaryl groups of formula (VA), (VB) or (VC):

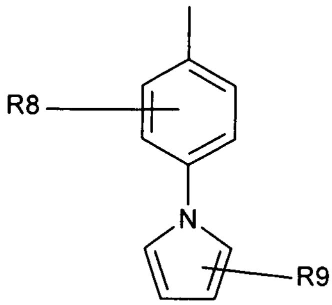

(VA)

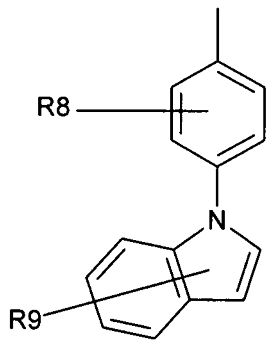

(VB)

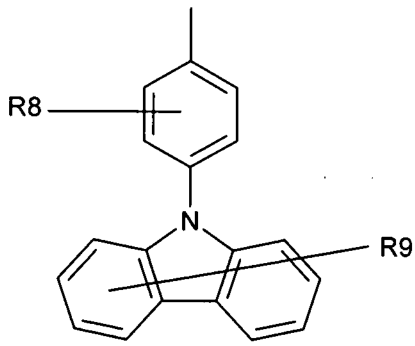

(VC)

in which $R_8$ and $R_9$ represent, independently of each other, a group chosen from (i) linear, branched or cyclic,

saturated or unsaturated hydrocarbon-based groups containing 1 to 30 carbon atoms, optionally comprising 1 to 5 hetero atoms chosen from N, O, S, Si and P, and/or optionally halogenated or perhalogenated; (ii) halogen atoms, ; (iii) - CN (nitrile), -COOH (carboxylate) or -$NO_2$ (nitro) groups; (iv) a hydrogen atom; (v) a group chosen from -C(O)$NR_2R_3$, -$NR_2R_3$, -$OR_4$ and -$SR_4$ with $R_2$, $R_3$ and $R_4$ having the meanings given above;

- (v) a linear, branched or cyclic, saturated or unsaturated hydrocarbon-based group containing 1 to 30 carbon atoms, optionally comprising 1 to 5 hetero atoms chosen from N, O, S, Si and P; and especially a group chosen from -$C_6H_4$-$CONR_2R_3$, -$C_6H_4$-$NR_2R_3$ and -$C_6H_4$-$OR_4$ with $R_2$, $R_3$ and $R_4$ having the meanings given above;

- $R_7$ represents a group chosen from:

- (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated;

- (ii) halogen atoms;

- (iii) -CN (nitrile), -COOH (carboxylate), -$NO_2$ (nitro), -N=N- (azo), =NH (imino) or -$CONH_2$ (amide) groups;

- (iv) a hydrogen atom;

- (v) a group chosen from -C(O)$NR_2R_3$, -$NR_2R_3$, -$OR_4$ and -$SR_4$ with $R_2$, $R_3$ and $R_4$ having the meanings given above;

- (vi) the radical $R_7$ also possibly forming, with one of the bonds "i", "j", "k" or "g,h" taken with the radical $R_1$, or "f" taken with the radical $R_1$, a saturated hydrocarbon-based ring containing in total 3 to 8 carbon atoms, optionally comprising 1 to 5 hetero atoms chosen from N, O, S, Si and P;

- $R'_1$ represents a group chosen from:

- (i) a hydrogen atom;

- (ii) a linear, branched or cyclic, saturated or unsaturated hydrocarbon-based group containing 1 to 30 carbon atoms, optionally comprising 1 to 5 hetero atoms chosen from N, O, S, Si and P, and/or optionally halogenated or perhalogenated;

- (iii) a group chosen from -C(O)$NR_2R_3$, -$NR_2R_3$, -$OR_4$ and -$SR_4$, with $R_2$, $R_3$ and $R_4$ having the meanings given above;

- $R'_2$ represents a group chosen from:

- (i) linear, branched or cyclic, saturated or unsaturated hydrocarbon-based groups containing 1 to 30 carbon atoms, optionally comprising 1 to 5 hetero atoms chosen from N, O, S, Si and P, and/or optionally halogenated or perhalogenated;

- (ii) halogen atoms;

- (iii) -CN (nitrile), -COOH (carboxylate), -$NO_2$ (nitro), -N=N- (azo), =NH (imino) or -$CONH_2$ (amide) groups;

- (iv) a hydrogen atom;

- (v) a group chosen from -C(O)$NR_2R_3$, -$NR_2R_3$, -$OR_4$ and -$SR_4$, with $R_2$, $R_3$ and $R_4$ having the meanings given above ; the composition comprising from 0,001 % to 20 % by weight of the said photochromic dye, relative to the total weight of the cosmetic composition, and ΔE parameter being determined according to the protocol and with the help of the reference composition which are described in the specification, and is calculated according to :

$$\Delta E = [(L-L_0)^2 + (a-a_0)^2 + (b-b_0)^2]^{1/2}$$

wherein ($L_0$, $a_0$ and $b_0$) represent the initial trichromatic coordinates of the reference composition before irradiation and (L, a and b) represent the obtained coordinates after stopping the irradiation.

2. Composition according to claim 1, **characterized in that** the said photochromic die is present in an amount that is effective to give the said composition a dynamic effect in terms of colour and radiance.

3. Composition according to any one of the preceding claims, **characterized in that** the said naphthopyran derivative corresponds to formula (Ia) or (IIa) below:

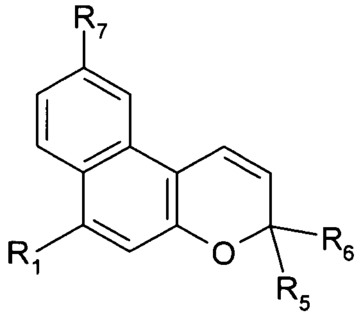

(Ia)

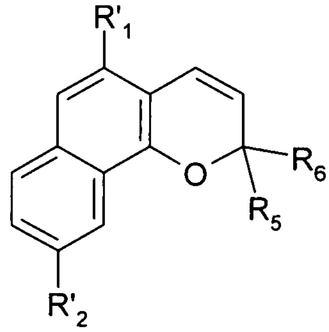

(IIa)

in which $R_1$, $R_5$, $R_6$, $R_7$, $R'_1$ and $R'_2$ are as defined in claim 1.

**4.** Composition according to any one of the preceding claims, **characterized in that** $R_1$ represents a hydrogen atom; a hydrocarbon-based ring with one of the bonds "f" or "gh" and the radical $R_7$; or a group chosen from $-COOR_4$, $-NR_2R_3$, $-OR_4$ and $-SR_4$, in which:

- $R_2$ and $R_3$ either may represent, independently of each other, a linear, branched or cyclic, saturated or unsaturated hydrocarbon-based group containing 1 to 20 carbon atoms, optionally comprising 1 to 5 hetero atoms chosen from N, O, S, Si and P,
or, taken together with the nitrogen atom to which they are attached, may form a saturated or unsaturated hydrocarbon-based heterocycle containing 3 to 10 carbon atoms and optionally 1 to 5 other hetero atoms chosen from N, O, S, Si and P, the said ring optionally being substituted with at least one linear, branched or cyclic, saturated or unsaturated hydrocarbon-based radical containing 1 to 20 carbon atoms, optionally comprising 1 to 5 hetero atoms chosen from N, O, S, Si and P;
- $R_4$ may represent a linear, branched or cyclic, saturated or unsaturated hydrocarbon-based group containing 1 to 20 carbon atoms, optionally halogenated or perhalogenated, and/or optionally comprising 1 to 5 hetero atoms chosen from N, O, S, Si and P;
and/or
- $R_5$ and $R_6$ may represent, independently of each other, a group chosen from:
- the saturated cyclic aminoaryl groups of formula (IIA) or (IIB):

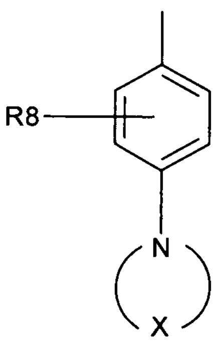

(IIA)

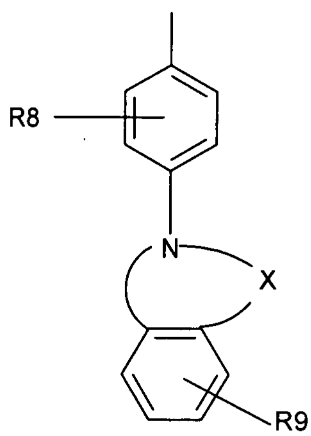

(IIB)

in which the ring comprising N and X is a saturated ring which contains in total 3 to 30, including nitrogen, the rest being carbon atoms and/or hetero atoms chosen from O, S, Si and P and/or groups chosen from -NH and -NR with R representing a linear, branched or cyclic, saturated or unsaturated hydrocarbon-based radical containing 1 to 20 carbon atoms, optionally comprising 1 to 5 hetero atoms chosen from N, O, S, Si and P;
- a linear, branched or cyclic, saturated or unsaturated hydrocarbon-based group containing 1 to 30 carbon atoms, optionally comprising 1 to 5 hetero atoms chosen from N, O, S, Si and P; and especially a group chosen from $-C_6H_4-CONR_2R_3$, $-C_6H_4-NR_2R_3$ and $-C_6H_4-OR_4$ with $R_2$, $R_3$ and $R_4$ having the meanings given above.

5. Composition according to any one of the preceding claims, **characterized in that** the said photochromic dye corresponds to formula (I) or (Ia) for which:

- $R_1$ represents hydrogen; or a group -COOR with R being a saturated hydrocarbon-based radical containing 1 to 12 or a group

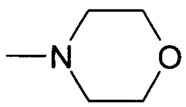

and/or
- $R_5$ and $R_6$ represent, independently of each other, either (i) a group of formula (IIA):

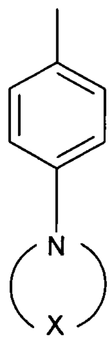

(IIA)

in which the ring comprising N and X is a saturated ring containing in total 4 to 7 atoms including nitrogen, and especially 3 to 5 carbon atoms and 0 or 1 oxygen atom; and in particular a group of formula:

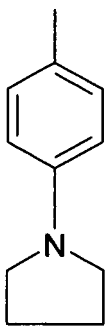

or

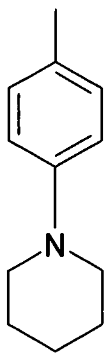

or

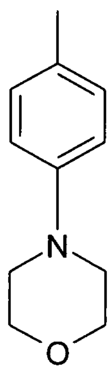

or (ii) a linear, branched or cyclic, saturated or unsaturated hydrocarbon-based group containing 5 to 14 carbon atoms, optionally comprising 1 or 2 hetero atoms chosen from N, O or S;

and/or
$R_7$ represents a hydrogen atom or a group $-NR_2R_3$, with $R_2$ and $R_3$ representing, independently of each other, a linear or branched, saturated hydrocarbon-based group containing 1 to 12 carbon atoms.

**6.** Composition according to any one of the preceding claims, **characterized in that** the said photochromic dye corresponds to formula (II) or (IIa) for which:

- $R'_1$ represents hydrogen or a group -COOR with R being a saturated hydrocarbon-based radical containing 1 to 12 carbon atoms;
and/or
- $R_5$ and $R_6$ represent, independently of each other, either (i) a group of formula (IIA):

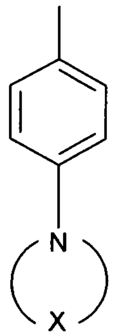

(IIA)

in which the ring comprising N and X is a saturated ring containing in total 4 to 7 atoms including nitrogen, or (ii) a linear, branched or cyclic, saturated or unsaturated hydrocarbon-based group containing 5 to 14 carbon atoms, optionally comprising 1 or 2 hetero atoms chosen from N, O and S;
and/or
- $R'_2$ represents hydrogen or a group -NR'R", with R' and R", which may be identical or different, representing a linear or branched, saturated hydrocarbon-based group containing 1 to 12 carbon atoms.

**7.** Composition according to any one of the preceding claims, **characterized in that** the said photochromic dye is chosen from:

- 3,3-di(4-methoxyphenyl)-6-morpholino-3H-naphtho[2,1-b]pyran
- 3-phenyl-3-(4-morpholinophenyl)-6-morpholino-3H-naphtho[2,1-b]pyran
- 3-phenyl-3-(4-piperidinophenyl)-6-morpholino-3H-naphtho[2,1-b]pyran
- 3-phenyl-3-(4-piperidinophenyl)-6-carboxymethyl-9-N-dimethyl-3H-naphtho[2,1-b]pyran
- 2-phenyl-2-(4-piperidinophenyl)-5-carboxymethyl-9-N-dimethyl-2H-naphtho[1,2-b]pyran, and mixtures thereof.

**8.** Composition according to any one of the preceding claims, **characterized in that** the said photochromic dye comprises at least two different naphthopyran derivatives.

**9.** Composition according to any one of the preceding claims, **characterized in that** it combines with the said photochromic dye at least a second dye.

**10.** Composition according to claim 9, **characterized in that** the second dye is chosen from goniochromatic colouring agents, water-soluble or liposoluble monochromatic dyes, pigments, reflective particles and nacres, and mixtures thereof.

**11.** Composition according to any one of the preceding claims, **characterized in that** it comprises, as second dye, at least one goniochromatic colouring agent.

**12.** Composition according to claim 11, **characterized in that** it comprises from 0.1 % to 60% by weight of the said goniochromatic colouring agent, relative to the total weight of the composition.

**13.** Composition according to claim 11 or 12, **characterized in that** the goniochromatic colouring agent is chosen from multilayer interference structures and liquid-crystal colouring agents.

**14.** Composition according to claim 13, **characterized in that** the goniochromatic colouring agent of multilayer interference structure comprises at least two layers, each layer, optionally independently of the other layer(s), being made from at least one material chosen from the group consisting of the following materials: $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolite, alloys and polymers, and combinations thereof.

**15.** Composition according to claim 13, **characterized in that** the liquid-crystal colouring agent is obtained by polymerization of a monomer mixture comprising:

- a) at least a first monomer A of formula (I) Y1-A1-M1-A2-Y2
in which

- i) Y1 and Y2, which may be identical or different, represent an acrylate or methacrylate group;
- ii) A1 and A2, which may be identical or different, represent a group of formula $-CnH_2n-$, in which n is an integer ranging from 1 to 20;
- iii) M1 denotes a group of general formula (I') $-R_1-X_1-R_2-X_2-R_3-X_3-R_4-$, in which $R_1$ and $R_4$ denote -O-, and $R_2$ and $R_3$ denote -COO-,
and $X_1$, $X_2$ and $X_3$ are a 1,4-phenylene group, the carbonyl group -CO- of $R_2$ and of $R_3$, respectively, being linked to the group $X_1$ or $X_3$, respectively,
and

- b) at least a second monomer B, which is chiral, of formula (II) V1- W1- Z- W2- V2, in which

- i) V1 denotes an acrylate or methacrylate group, and V2 denotes a $C_1$-$C_{20}$ alkyl, $C_1$-$C_{20}$ alkoxy, ($C_1$-$C_{20}$) alkoxycarbonyl or -OH group;
- ii) W1 represents a divalent group of formula -X' 1-CO-O-,

W2 represents a divalent group of formula -O-CO-X' 1-,
in which X'1 denotes a 1,4-phenylene group,
and Z denotes a chiral group containing two bonds, derived from the dianhydrohexite group, in particular a divalent radical of formula:

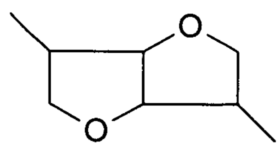

**16.** Composition according to claim 15, **characterized in that** the liquid-crystal colouring agent is obtained from a monomer mixture comprising from 70% to 99% by weight of monomer A and from 1% to 30% by weight of monomer B, relative to the total weight of monomer A and of monomer B.

**17.** Composition according to claim 13, 15 or 16, **characterized in that** the liquid-crystal colouring agent is present in a content ranging from 0.01% to 99% by weight, relative to the total weight of the composition.

**18.** Composition according to any one of the preceding claims, **characterized in that** it comprises, as second dye, from 0.001% to 15% by weight of at least one liposoluble or water-soluble dye relative to the total weight of the composition.

**19.** Composition according to any one of the preceding claims, **characterized in that** it comprises, as second dye, from 0.01% to 25% by weight of at least one pigment relative to the total weight of the composition.

**20.** Composition according to any one of the preceding claims, **characterized in that** it comprises, as second dye, from 0.01% to 20% by weight of nacres relative to the total weight of the composition.

**21.** Composition according to any one of the preceding claims, **characterized in that** it comprises, as second dye, from 0.1% to 20% of reflective particles relative to the total weight of the composition.

**22.** Composition according to any one of the preceding claims, **characterized in that** it comprises at least one oily phase.

**23.** Composition according to claim 22, **characterized in that** the oily phase comprises one or more polar or apolar, volatile or non-volatile.

**24.** Composition according to claim 22 or 23, **characterized in that** the oily phase comprises from 5% to 100% by weight of polar oils relative to the total weight of the said oily phase.

**25.** Composition according to claim 22, 23 or 24, **characterized in that** the oily phase comprises from 5% to 100% by weight apolar oils relative to the total weight of the said oily phase.

**26.** Composition according to any one of claims 22 to 25, **characterized in that** the oils may be chosen from volatile or non-volatile oils of animal, plant, mineral or synthetic origin, and mixtures thereof.

**27.** Composition according to claim 26, **characterized in that** the oils are chosen from animal or plant oils, synthetic esters and ethers, especially of fatty acids, fatty alcohols, linear or branched hydrocarbons of mineral or synthetic origin, and glycerides, and mixtures thereof.

**28.** Composition according to any one of claims 22 to 27, **characterized in that** the oily phase is such that the photochromic dye(s) is(are) soluble or dispersible therein.

**29.** Composition according to any one of claims 22 to 28, **characterized in that** it comprises from 1% to 99% by weight of oily phase relative to the total weight of the said composition.

**30.** Composition according to any one of the preceding claims, **characterized in that** it comprises from 0.1 % to 50% by weight of a fatty substance other than an oil, relative to the total weight of the said composition.

**31.** Composition according to any one of the preceding claims, **characterized in that** it is anhydrous.

**32.** Composition according to any one of claims 1 to 30, **characterized in that** it comprises at least one aqueous phase.

**33.** Composition according to claim 32, **characterized in that** the said aqueous phase comprises from 0.1 % to 14% by weight of a $C_2$-$C_6$ monoalcohol, relative to the total weight of the said aqueous phase.

**34.** Composition according to any one of the preceding claims, **characterized in that** it comprises at least one surfactant.

**35.** Composition according to claim 34, **characterized in that** said surfactant is present in an amount from 0.01% to 30% by weight relative to the total weight of the composition.

**36.** Composition according to any one of the preceding claims, **characterized in that** it comprises at least one thickener.

**37.** Composition according to claim 36, **characterized in that** said thickener is present in an amount from 0.01% to 6% by weight relative to the total weight of the said composition.

**38.** Composition according to any one of the preceding claims, **characterized in that** it comprises at least one film-forming polymer.

**39.** Composition according to claim 38, **characterized in that** said film-forming polymer is present in an amount from 0.01 % to 4% by weight relative to the total weight of the composition.

**40.** Composition according to any one of the preceding claims, **characterized in that** it comprises at least one filler.

**41.** Composition according to claim 40, **characterized in that** said filler is present in a proportion of from 0.01% to 60% by weight relative to the total weight of the said composition.

**42.** Process for making up the skin, the lips and/or the integuments, comprising the application of at least one composition according to any one of the preceding claims to the skin, the lips and/or the integuments.

**43.** Use of at least one photochromic dye with a difference in hue ΔE at least equal to 5, and being a naphtopyran derivative of formula (I) or (II) as defined according to any one of claims 1 to 8, in a cosmetic composition, especially for making up the skin, the integuments, the nails and the lips, containing at least one second dye, to reversibly change colour when they are subjected to a radiation containing ultraviolet rays, the said composition comprising from 0,001 to 20 % by weight of the said photochromic dye, relative to the total weight of the composition.

**Patentansprüche**

**1.** Kosmetische Zusammensetzung, die in einem physiologisch verträglichen Medium mindestens zwei Farbstoffe einschließt, wovon mindestens ein Farbstoff photochrom ist, **dadurch gekennzeichnet, dass** der photochrome Farbstoff eine Gesichtsfarbentoleranz ΔE von mindestens gleich 5 aufweist und mindestens ein Naphthopyran-Derivat ist, das in der Zusammensetzung in einer gelösten Form vorhanden und vom Typ eines 2H-Naphtho-[2,1-b]-pyrans der Formel (I) oder vom Typ eines 3H-Naphtho-[2,1-b]-pyrans der Formel (II) ist:

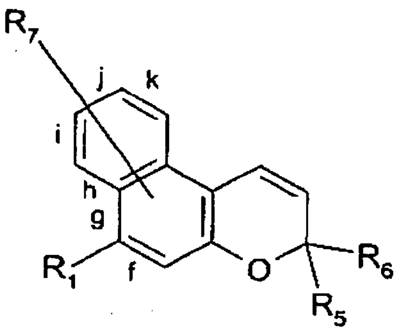

(I)

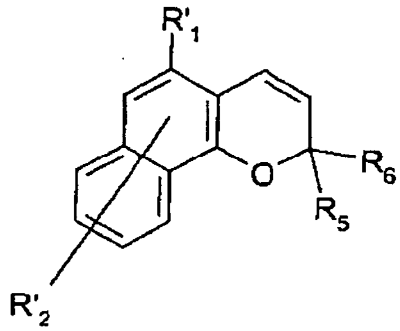

(II)

wobei

- R1 Folgendes darstellt:

  - (i) ein Wasserstoffatom;
  - (ii) eine Kohlenwasserstoffgruppe mit 1 bis 30 linearen, verzweigten oder cyclischen gesättigten oder ungesättigten Kohlenstoffatomen, die gegebenenfalls 1 bis 5 Heteroatome einschließt, die aus N, O, S, Si und P ausgewählt sind, und/oder die gegebenenfalls halogeniert oder perhalogeniert ist;
  - (iii) ein Kohlenwasserstoffcyclus, der mit der einen der Bindungen "f" oder "gh" und dem Rest $R_7$ gebildet ist; oder
  - (iv) eine Gruppe, die aus $-COOR_4$, $-C(O)NR_2R_3$, $-NR_2R_3$, $-OR_4$ und $-SR_4$ ausgewählt ist, wobei:

    - $R_2$ und $R_3$, unabhängig voneinander, entweder eine Kohlenwasserstoffgruppe mit 1 bis 20 linearen, verzweigten oder cyclischen, gesättigten oder ungesättigten Kohlenstoffatomen, die gegebenenfalls 1 bis 5 Heteroatome, ausgewählt unter N, O, S, Si und P, einschließt, darstellen, oder zusammengenommen mit dem Stickstoffatom, an das sie gebunden sind, einen gesättigten oder ungesättigten Kohlenwasserstoff-Heterocyclus bilden, der 3 bis 10 Kohlenstoffatome und gegebenenfalls 1 bis 5 Heteroatome, die aus N, O, S, Si und P ausgewählt sind, einschließt, wobei der Cyclus gegebenenfalls mit mindestens einem Kohlenwasserstoffrest mit 1 bis 20 mit linearen, verzweigten oder cyclischen, gesättigten oder ungesättigten Kohlenstoffatomen substituiert ist und gegebenenfalls 1 bis 5 Heteroatome, ausgewählt aus N, O, S, Si und P, einschließt;
    - $R_4$ eine lineare, verzweigte oder cyclische, gesättigte oder ungesättigte, gegebenenfalls halogenierte oder perhalogenierte Kohlenwasserstoffgruppe mit 1 bis 20 Kohlenstoffatomen ist, und/oder die gegebenenfalls 1 bis 5 Heteroatome, aus N, O, S, Si und P ausgewählt sind, umfasst;
- $R_5$ und $R_6$, unabhängig voneinander, eine Gruppe darstellen, die ausgewählt ist aus:

- (i) den cyclischen gesättigen Aminoarylgruppen der Formel (IIA) oder (IIB):

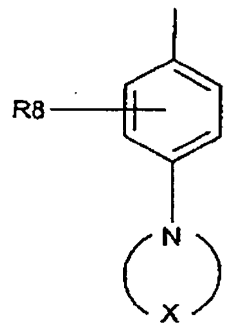

(IIA)

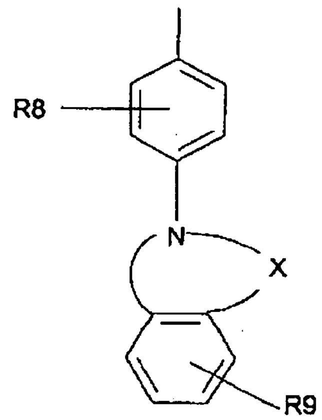

(IIB)

wobei der Cyclus, der N und X umfasst, ein gesättigter Cyclus ist, der insgesamt 3 bis 30 Atome, einschließlich Stickstoff, umfasst, wobei der Rest Kohlenstoff- und/oder Heteroatome, die aus O, S, Si, P ausgewählt sind, und/oder Gruppierungen darstellt, die aus -NH und -NR ausgewählt sind, wobei R ein Kohlenwasserstoffrest mit 1 bis 20 linearen, verzweigten oder cyclischen, gesättigten oder ungesättigten Kohlenstoffatomen ist, der gegebenenfalls 1 bis 5 Heteroatome umfasst, die aus N, O, S, Si und P ausgewählt sind;
- (ii) den Indolinoarylgruppen der Formel (III):

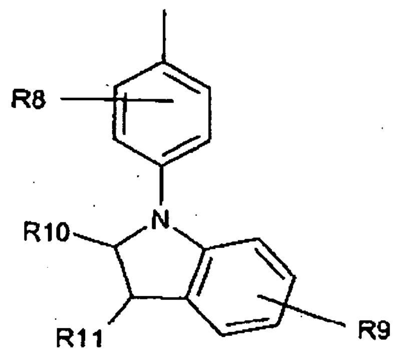

(III)

wobei $R_{10}$ und $R_{11}$, unabhängig voneinander, eine Gruppe darstellen, die ausgewählt ist aus (i) den Kohlenwasserstoffgruppen mit 1 bis 30 Kohlenstoffatomen, die linear, verzweigt oder cyclisch, gesättigt oder ungesättigt sind, die gegebenenfalls 1 bis 5 Heteroatome einschließt, die aus N, O, S, Si und P ausgewählt sind, und/oder gegebenenfalls halogeniert oder perhalogeniert sind; (ii) den Halogenatomen; (iii) den Gruppen -CN (Nitril); -COOH (Carboxylat); $-NO_2$ (Nitro); (iv) einem Wasserstoffatom; (v) einer Gruppe, die aus $-C(O)NR_2R_3$, $-NR_2R_3$, $-OR_4$ oder $-SR_4$ ausgewählt ist, wobei $R_2$, $R_3$ und $R_4$ die vorstehend gegebenen Bedeutungen besitzen; (vi) den Resten $R_{10}$ und $R_{11}$, die zusammen einen gesättigten oder ungesättigten Kohlenwasserstoffcyclus mit insgesamt 5 bis 8 Atomen (einschließlich der Atome des Indolin-Cyclus) bilden können, wobei die Atome

ausgewählt sind aus C, O, S und/oder NR, wobei R H oder ein Kohlenwasserstoffrest mit 1 bis 20 linearen, verzweigten oder cyclischen, gesättigten oder ungesättigten Kohlenstoffatomen mit 1 bis 20 Kohlenstoffatomen ist, der gegebenenfalls 1 bis 5 Heteroatome einschließt, die aus N, O, S, Si und P ausgewählt sind,

- (iii) den Gruppen der Formel (IV):

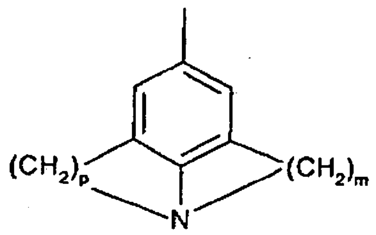

(IV)

wobei m und p, unabhängig voneinander, ganze Zahlen von 2 bis 5 sind;

- (iv) den cyclischen ungesättigten Aminoarylgruppen der Formeln (VA), (VB) oder (VC):

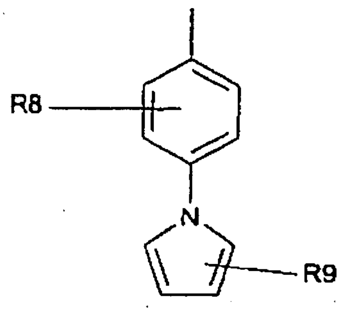

(VA)

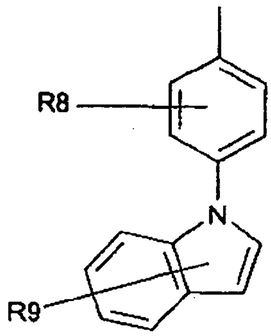

(VB)

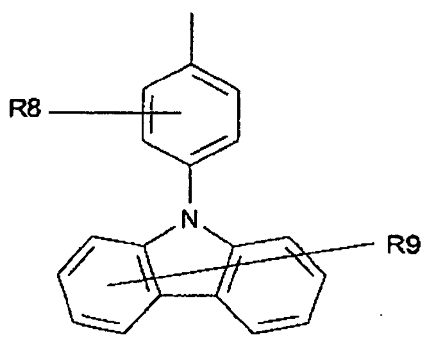

(VC)

wobei $R_8$ und $R_9$, unabhängig voneinander, eine Gruppe darstellen, die ausgewählt ist aus (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome umfassen, die aus N, O, S, Si und P ausgewählt sind, und/oder gegebenenfalls halogeniert oder perhalogeniert sind; (ii)

den Halogenatomen; (iii) den Gruppen -CN (Nitril), -COOH (Carboxylat), $-NO_2$ (Nitro); (iv) einem Wasserstoffatom, (v) einer Gruppe, ausgewählt aus $-C(O)NR_2R_3$, $-NR_2R_3$, $-OR_4$ oder $-SR_4$, wobei $R_2$, $R_3$ und $R_4$ die vorstehend gegebenen Bedeutungen besitzen;
- (v) einer linearen, verzweigten oder cyclischen, gesättigten oder ungesättigten Kohlenwasserstoffgruppe mit 1 bis 30 Kohlenstoffatomen, die 1 bis 5 Heteroatome, ausgewählt aus N, O, S, Si und P, umfasst; und insbesondere einer Gruppe, die aus $-C_6H_4-CONR_2R_3$, $-C_6H_4-NR_2R_3$ und $-C_6H_4-OR_4$ ausgewählt ist, wobei $R_2$, $R_3$ und $R_4$ die vorstehend gegebenen Bedeutungen besitzen;

- $R_7$ eine Gruppe darstellt, ausgewählt aus:

- (i) den linearen, verzweigten oder cyclischen, gesättigten oder ungesättigten Kohlenwasserstoffgruppen mit 1 bis 30 Kohlenstoffatomen, die gegebenenfalls 1 bis 5 Heteroatome einschließen, die aus N, O, S, Si und P ausgewählt sind, und/oder gegebenenfalls halogeniert oder perhalogeniert sind;
- (ii) den Halogenatomen;
- (iii) den Gruppen -CN (Nitril), -COOH (Carboxylat), $-NO_2$ (Nitro); -N=N-(Azo); =NH (Imino); $-CONH_2$ (Amid);
- (iv) einem Wasserstoffatom;
- (v) einer Gruppe, die aus $-C(O)NR_2R_3$, $-NR_2R_3$, $-OR_4$ oder $-SR_4$ ausgewählt ist, wobei $R_2$, $R_3$ und $R_4$ die vorstehend gegebenen Bedeutungen besitzen;
- (vi) dem Rest $R_7$, der ferner mit der einen der Bindungen "i", "j", "k" oder "gh", zusammen mit dem Rest $R_1$, oder mit "f", zusammen mit dem Rest $R_1$, einen gesättigten Kohlenwasserstoffcyclus mit insgesamt 3 bis 8 Kohlenstoffatomen bilden kann, der gegebenenfalls 1 bis 5 Heteroatome einschließt, die aus N, O, S, Si und P ausgewählt sind;

- $R'_1$ eine Gruppe darstellt, die ausgewählt ist aus:

- (i) einem Wasserstoffatom;
- (ii) einer linearen, verzweigten oder cyclischen, gesättigten oder ungesättigten Kohlenwasserstoffgruppe mit 1 bis 30 Kohlenstoffatomen, die gegebenenfalls 1 bis 5 Heteroatome umfasst, die aus N, O, S, Si und P ausgewählt sind, und/oder die gegebenenfalls halogeniert oder perhalogeniert ist;
- (iii) einer Gruppe, die aus $-C(O)NR_2R_3$, $-NR_2R_3$, $-OR_4$ und $SR_4$ ausgewählt ist, wobei $R_2$, $R_3$ und $R_4$ die vorstehend gegebenen Bedeutungen besitzen;

- $R'_2$ eine Gruppe darstellt, die ausgewählt ist aus:

- (i) den linearen, verzweigten oder cyclischen, gesättigten oder ungesättigten Kohlenwasserstoffgruppe mit 1 bis 30 Kohlenstoffatomen, die gegebenenfalls 1 bis 5 Heteroatome umfassen, die aus N, O, S, Si und P ausgewählt sind, und/oder die gegebenenfalls halogeniert oder perhalogeniert ist;
- (ii) den Halogenatomen;
- (iii) den Gruppen -CN (Nitril), -COOH (Carboxylat), $-NO_2$ (Nitro); -N=N- (A-zo); =NH (Imino); $-CONH_2$ (Amid);
- (iv) einem Wasserstoffatom;
- (v) einer Gruppe, die ausgewählt ist aus $-C(O)NR_2R_3$, $-NR_2R_3$, $-OR_4$ oder $-SR_4$, wobei $R_2$, $R_3$ und $R_4$ die vorstehend gegebenen Bedeutungen besitzen;

wobei die Zusammensetzung, bezogen auf das Gesamtgewicht der Zusammensetzung, 0,001 bis 20 Gew.-% des photochromen Farbstoffes umfasst und der Parameter ΔE nach dem Protokoll und mit Hilfe der Kontrollzusammensetzung, die in der Beschreibung beschrieben ist, bestimmt und berechnet wird nach:

$$\Delta E = [(L - L_0)^2 + (a - a_0)^2 + (b - b_0)^2]^{1/2}$$

wobei ($L_0$, $a_0$ und $b_0$) die trichromatischen Ausgangskoordinaten der Kontrollzusammensetzung vor der Bestrahlung darstellen und (L, a und b) die nach Anhalten der Bestrahlung erhaltenen Koordinaten darstellen.

2. Zusammensetzung nach Anspruch 1, **dadurch gekennzeichnet**, das der photochrome Farbstoff in einer wirksamen Menge vorhanden ist, um eine dynamische Wirkung hinsichtlich Farbe und Toleranz der Zusammensetzung zu

verleihen.

**3.** Zusammensetzung nach Anspruch 1 oder 2, **dadurch gekennzeichnet, dass** das Naphthopyran-Derivat den folgenden Formeln (Ia) oder (IIa) gehorcht,

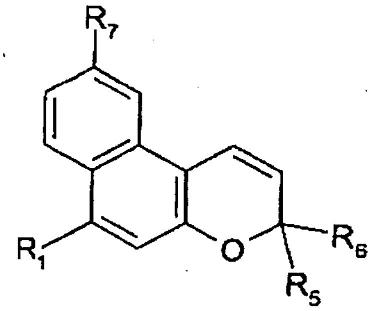

(Ia)

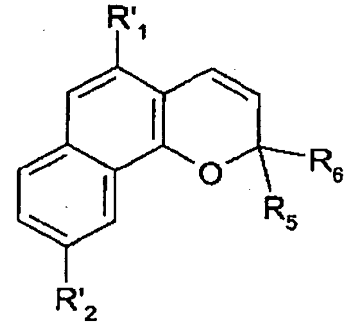

(IIa)

wobei $R_1$, $R_5$, $R_6$, $R_7$, $R_1$' und $R_2$' wie in Anspruch 1 definiert sind.

**4.** Zusammensetzung nach einem der vorhergehenden Ansprüche, **dadurch gekennzeichnet, dass** $R_1$ ein Wasserstoffatom, einen Kohlenwasserstoffcyclus mit einer der Bindungen "f" oder "gh" und dem Rest $R_7$ oder eine Gruppe darstellt, die aus $-COOR_4$, $-NR_2R_3$, $-OR_4$ oder $-SR_4$ ausgewählt ist, wobei

- $R_2$ und $R_3$, unabhängig voneinander, entweder eine Kohlenwasserstoffgruppe mit 1 bis 20 linearen, verzweigten oder cyclischen, gesättigten oder ungesättigten Kohlenstoffatomen darstellen, die gegebenenfalls 1 bis 5 Heteroatome einschließt, die aus N, O, S, Si und P ausgewählt sind,
oder zusammengenommen mit dem Stickstoffatom, an das sie gebunden sind, einen gesättigten oder ungesättigten Kohlenwasserstoff-Heterocyclus bilden können, der 3 bis 10 Kohlenstoffatome und gegebenenfalls 1 bis 5 Heteroatome, ausgewählt aus N, O, S, Si und P, einschließt, wobei der Cyclus gegebenenfalls mit mindestens einem Kohlenwasserstoffrest mit 1 bis 20 mit linearen, verzweigten oder cyclischen, gesättigten oder ungesättigten Kohlenstoffatomen substituiert ist und gegebenenfalls 1 bis 5 Heteroatome, ausgewählt aus N, O, S, Si und P, einschließt;
- $R_4$ eine lineare, verzweigte oder cyclische, gesättigte oder ungesättigte, gegebenenfalls halogenierte oder perhalogenierte Kohlenwasserstoffgruppe mit 1 bis 20 Kohlenstoffatomen darstellen kann, und/oder die gegebenenfalls 1 bis 5 Heteroatome umfasst, die ausgewählt sind aus N, O, S, Si und P;
und/oder
- $R_5$ und $R_6$, unabhängig voneinander, eine Gruppe darstellen, die ausgewählt ist aus:

  - den cyclischen gesättigen Aminoarylgruppen der Formel (IIA) oder (IIB):

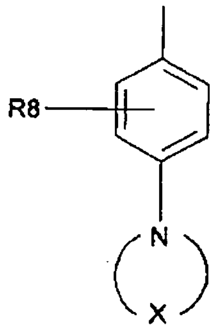

(IIA)

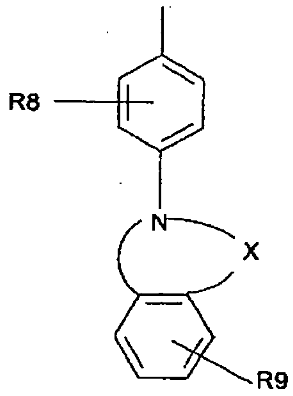

(IIB)

wobei der Cyclus, der N und X umfasst, ein gesättigter Cyclus ist, der insgesamt 3 bis 30 Atome, einschließlich Stickstoff, umfasst, wobei der Rest Kohlenstoff- und/oder Heteroatome, die aus O, S, Si, P ausgewählt sind, und/oder Gruppierungen darstellt, die aus -NH und -NR ausgewählt sind, wobei R ein Kohlenwasserstoffrest mit 1 bis 20 linear verzweigten oder cyclischen, gesättigten oder ungesättigten Kohlenstoffatomen ist, der gegebenenfalls 1 bis 5 Heteroatome umfasst, die aus N, O, S, Si und P ausgewählt sind;

- einer linearen, verzweigten oder cyclischen, gesättigten oder ungesättigen Kohlenwasserstoffgruppe mit 1 bis 30 Kohlenstoffatomen, die gegebenenfalls 1 bis 5 Heteroatome, ausgewählt aus N, O, S, Si und P; und insbesondere aus einer Gruppe, ausgewählt aus $-C_6H_4CONR_2R_3$, $-C_6H_4NR_2R_3$ und $C_6H_4-OR_4$, wobei $R_2$, $R_3$ und $R_4$ die vorstehend gegebenen Bedeutungen besitzen.

**5.** Zusammensetzung nach einem der vorhergehenden Ansprüche, **dadurch gekennzeichnet, dass** der photochrome Farbstoff der Formel (I) oder (Ia) gehorcht, für die:

- $R_1$ Wasserstoff oder eine Gruppe -COOR darstellt, wobei R ein gesättigter Kohlenwasserstoffrest mit 1 bis 12 Kohlenstoffatomen ist; oder eine Gruppe

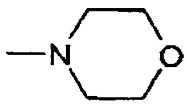

darstellt, und/oder
- $R_5$ und $R_6$, unabhängig voneinander, entweder (i) eine Gruppe der Formel (IIA):

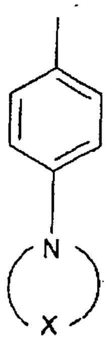

(IIA)

wobei der Cyclus, der N und X umfasst, ein gesättigter Cyclus ist, der insgesamt 4 bis 7 Atome, einschließlich Stickstoff, umfasst, und insbesondere 3 bis 5 Kohlenstoffatome oder 0 oder 1 Sauerstoffatom umfasst; und

insbesondere eine Gruppe der Formel darstellt:

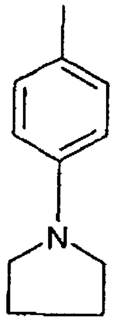 oder 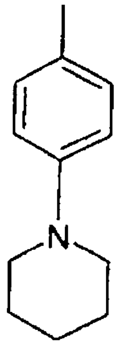 oder 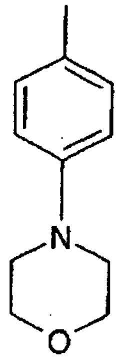

oder (ii) eine lineare, verzweigte oder cyclische, gesättigte oder ungesättigte Kohlenwasserstoffgruppe mit 5 bis 14, insbesondere 6 bis 10 Kohlenstoffatomen, die gegebenenfalls 1 bis 2 Heteroatome, ausgewählt aus N, O oder S, darstellt; und/oder

- $R_7$ ein Wasserstoffatom oder eine Gruppe $-NR_2R_3$ darstellt, wobei $R_2$ und $R_3$, unabhängig voneinander, eine gesättigte, lineare oder verzweigte Kohlenwasserstoffgruppe mit 1 bis 12 Kohlenstoffatomen darstellen.

**6.** Zusammensetzung nach einem der vorhergehenden Ansprüche, **dadurch gekennzeichnet, dass** der photochrome Farbstoff der Formel (II) oder (IIa) gehorcht, wobei:

- $R'_1$ Wasserstoff oder eine Gruppe -COOR darstellt, wobei R ein gesättigter Kohlenwasserstoffrest mit 1 bis 12 Kohlenstoffatomen ist;

und/oder

- $R_5$ und $R_6$, unabhängig voneinander, entweder (i) eine Gruppe der Formel (IIA):

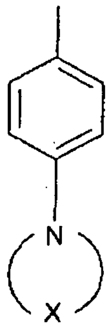 (IIA)

wobei der Cyclus N und X umfasst und ein gesättigter Cyclus ist, der insgesamt 4 bis 7 Atome, einschließlich Stickstoff, umfasst,

oder (ii) eine lineare, verzweigte oder cyclische, gesättigte oder ungesättigte Kohlenwasserstoffgruppe mit 5 bis 14 Kohlenstoffatomen, die gegebenenfalls 1 bis 2 Heteroatome, ausgewählt aus N, O oder S, umfasst;

und/oder

- $R'_2$ Wasserstoff oder eine Gruppe -NR'R" darstellt, wobei R' und R", gleich oder verschieden, eine gesättigte lineare oder verzweigte Kohlenwasserstoffgruppe mit 1 bis 12 Kohlenstoffatomen darstellen.

**7.** Zusammensetzung nach einem der vorhergehenden Ansprüche, **dadurch gekennzeichnet, dass** der photochrome Farbstoff ausgewählt ist aus

- 3,3-Di-(4-methoxyphenyl)-6-morpholino-3H-naphtho[2,1-b]pyran
- 3-Phenyl-3-(4-morpholinophenyl)-6-morpholino-3H-naphtho[2,1-b]pyran
- 3-Phenyl-3-(4-piperidinophenyl)-6-morpholino-3H-naphtho[2,1-b]pyran
- 3-Phenyl-3-(4-piperidinophenyl)-6-carboxymethyl-9-N-dimethyl-3H-naphtho[2,1-b]pyran

- 2-Phenyl-2-(4-piperidinoyphenyl)-5-carboxymethyl-9-N-dimethyl-2H-naphtho[2,1-b]pyran und ihren Gemischen.

**8.** Zusammensetzung nach einem der vorhergehenden Ansprüche, **dadurch gekennzeichnet, dass** der photochrome Farbstoff mindestens zwei distinkte Naphthopyran-Derivate umfasst.

**9.** Zusammensetzung nach einem der vorhergehenden Ansprüche, **dadurch gekennzeichnet, dass** sie mit dem photochromen Farbstoff mindestens einen zweiten Farbstoff kombiniert.

**10.** Zusammensetzung nach dem vorhergehenden Anspruch, **dadurch gekennzeichnet, dass** der zweite Farbstoff aus goniochromatischen Farbmitteln, wasser- und fettlöslichen monochromen Farbmitteln, Pigmenten, reflektierenden Teilchen, Perlmuttlacken und ihren Gemischen ausgewählt ist.

**11.** Zusammensetzung nach einem der vorhergehenden Ansprüche, **dadurch gekennzeichnet, dass** sie als zweiten Farbstoff mindestens ein goniochromatisches Farbmittel umfasst.

**12.** Zusammensetzung nach dem vorhergehenden Anspruch, **dadurch gekennzeichnet, dass** sie 0,1 bis 60 Gew.-% des goniochromatischen Farbmittels bezüglich des Gesamtgewichts der Zusammensetzung umfasst.

**13.** Zusammensetzung nach Anspruch 11 oder 12, **dadurch gekennzeichnet, dass** das goniochromatische Farbmittel aus mehrschichtigen Interferenzstrukturen und flüssigkristallinen Farbmitteln ausgewählt ist.

**14.** Zusammensetzung nach dem vorhergehenden Anspruch, **dadurch gekennzeichnet, dass** das goniochromatische Farbmittel mit mehrschichtiger Interferenzstruktur mindestens zwei Schichten umfasst, wobei jede Schicht unabhängig oder nicht von der (oder den) anderen Schicht(en) aus mindestens einem Material hergestellt ist, ausgewählt aus der Gruppe, bestehend aus den folgenden Materialien $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, Cryolith, Legierungen, Polymeren und ihren Kombinationen.

**15.** Zusammensetzung nach Anspruch 13, **dadurch gekennzeichnet, dass** das flüssigkristalline Farbmittel durch Polymerisation einer Mischung von Monomeren erhalten wird, umfassend:

- a) mindestens ein erstes Monomer A der Formel (I) Y1-A1-M1-A2-Y2, wobei

  - i) Y1 und Y2 gleich oder verschieden, eine Acrylat- oder Methacrylatgruppe darstellen;
  - ii) A1 und A2, gleich oder verschieden, eine Gruppe der Formel $-C_nH_{2n}-$ darstellen, wobei n eine ganze Zahl von 1 bis 20 ist;
  - iii) M1 eine Gruppe der allgemeinen Formel (I') $-R_1-X_1-R_2-X_2-R_3-X_3-R_4-$ darstellt, wobei $R_1$ und $R_4$ -O- bezeichnet, $R_2$ und $R_3$ -COO- bezeichnen und X1, X2, X3 eine 1,4-Phenylengruppe sind, wobei die Carbonylgruppe von $R_2$ bzw. $R_3$ jeweils an X1-, X3-Gruppen gebunden ist, und

- b) mindestens ein zweites chirales Monomer der Formel (II) V1-W1-Z-W2-V2, wobei

  - i) V1 eine Acrylat- oder Methacrylatgruppe bezeichnet, und V2 eine $C_1$-$C_{20}$-Alkylgruppe, eine $C_1$-$C_{20}$-Alkoxygruppe, eine Alkoxy-($C_1$-$C_{20}$)-carbonylgruppe, -OH bezeichnet;
  - ii) W1 eine zweiwertige Gruppe der Formel -X'-1-CO-O- bezeichnet,

W2 eine zweiwertige Gruppe der Formel -O-CO-X'-1- bezeichnet, wobei
X'1 eine 1,4-Phenylengruppe bezeichnet,
und Z eine chirale Gruppe mit zwei von der Dianhydrohexitgruppe ausgehenden Bindungen, insbesondere einen zweiwertigen Rest der Formel:

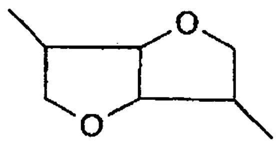

bezeichnet.

16. Zusammensetzung nach dem vorhergehenden Anspruch, **dadurch gekennzeichnet, dass** das flüssigkristalline Farbmittel aus einem Gemisch von Monomeren erhalten wird, umfassend 70 bis 99 Gew.-% von Monomer A und 1 bis 30 Gew.-% von Monomer B bezüglich des Gesamtgewichts von Monomer A und Monomer B.

17. Zusammensetzung nach Anspruch 13, 15 oder 16, **dadurch gekennzeichnet, dass** das flüssigkristalline Farbmittel in einem Gehalt von 0,01 bis 99 Gew.-% bezüglich des Gesamtgewichts der Zusammensetzung vorhanden ist.

18. Zusammensetzung nach einem der vorhergehenden Ansprüche, **dadurch gekennzeichnet, dass** sie als zweiten Farbstoff 0,001 bis 15 Gew.-% von mindestens einem fett- oder wasserlöslichen Farbmittel bezüglich des Gesamtgewichts der Zusammensetzung umfasst.

19. Zusammensetzung nach einem der vorhergehenden Ansprüche, **dadurch gekennzeichnet, dass** sie als zweiten Farbstoff 0,01 bis 25 Gew.-% von mindestens einem Pigment bezüglich des Gesamtgewichts der Zusammensetzung umfasst.

20. Zusammensetzung nach einem der vorhergehenden Ansprüche, **dadurch gekennzeichnet, dass** sie als zweiten Farbstoff 0,01 bis 20 Gew.-% Perlmuttlacke bezüglich des Gesamtgewichts der Zusammensetzung umfasst.

21. Zusammensetzung nach einem der vorhergehenden Ansprüche, **dadurch gekennzeichnet, dass** sie als zweiten Farbstoff 0,1 bis 20 % reflektierende Teilchen bezüglich des Gesamtgewichts der Zusammensetzung umfasst.

22. Zusammensetzung nach einem der vorhergehenden Ansprüche, **dadurch gekennzeichnet, dass** sie mindestens eine ölige Phase umfasst.

23. Zusammensetzung nach dem vorhergehenden Anspruch, **dadurch gekennzeichnet, dass** die ölige Phase ein oder mehrere, polare oder apolare, flüchtige oder nichtflüchtige Öle umfasst.

24. Zusammensetzung nach Anspruch 22 oder 23, **dadurch gekennzeichnet, dass** die ölige Phase 5 bis 100 Gew.-% polare Öle bezüglich des Gesamtgewichts der öligen Phase umfasst.

25. Zusammensetzung nach Anspruch 22, 23 oder 24, **dadurch gekennzeichnet, dass** die ölige Phase 5 bis 100 Gew.-% apolare Öle bezüglich des Gesamtgewichts der öligen Phase umfasst.

26. Zusammensetzung nach einem der Ansprüche 22 bis 25, **dadurch gekennzeichnet, dass** die Öle aus Ölen tierischen, pflanzlichen, mineralischen oder synthetischen Ursprungs, die flüchtig sind oder nicht, und aus ihren Gemischen ausgewählt werden können.

27. Zusammensetzung nach dem vorhergehenden Anspruch, **dadurch gekennzeichnet**, das sie aus tierischen oder pflanzlichen Ölen, den synthetischen Estern und Ethern, insbesondere von Fettsäuren, den Fettalkoholen, den linearen oder verzweigten Kohlenwasserstoffen mineralischen oder synthetischen Ursprungs, den Glyceriden und ihren Gemischen ausgewählt sind.

28. Zusammensetzung nach einem der Ansprüche 22 bis 27, **dadurch gekennzeichnet, dass** die ölige Phase so ist, dass der oder die photochromen Farbstoffe in ihr löslich oder dispergierbar sind.

29. Zusammensetzung nach einem der Ansprüche 22 bis 28, **dadurch gekennzeichnet, dass** sie 1 bis 99 Gew.-% ölige Phase bezüglich des Gesamtgewichts der Zusammensetzung umfasst.

**30.** Zusammensetzung nach einem der vorhergehenden Ansprüche, **dadurch gekennzeichnet, dass** sie 0,1 bis 50 Gew.-% eines Fettkörpers, der anders ist als ein Öl, bezüglich des Gesamtgewichts der Zusammensetzung umfasst.

**31.** Zusammensetzung nach einem der vorhergehenden Ansprüche, **dadurch gekennzeichnet, dass** sie wasserfrei ist.

**32.** Zusammensetzung nach einem der Ansprüche 1 bis 30, **dadurch gekennzeichnet, dass** sie mindestens eine wässrige Phase umfasst.

**33.** Zusammensetzung nach dem vorhergehenden Anspruch, **dadurch gekennzeichnet, dass** die wässrige Phase 0,1 bis 14 Gew.-% eines $C_2$-$C_6$-Monoalkohols bezüglich des Gesamtgewichts der wässrigen Phase umfasst.

**34.** Zusammensetzung nach einem der vorhergehenden Ansprüche, **dadurch gekennzeichnet, dass** sie mindestens ein Tensid umfasst.

**35.** Zusammensetzung nach dem vorhergehenden Anspruch, **dadurch gekennzeichnet, dass** das Tensid in einer Menge von 0,01 bis 30 Gew.-% bezüglich des Gewichts der Gesamtzusammensetzung vorhanden ist.

**36.** Zusammensetzung nach einem der vorhergehenden Ansprüche, **dadurch gekennzeichnet, dass** sie mindestens ein Verdickungsmittel umfasst.

**37.** Zusammensetzung nach dem vorhergehenden Anspruch, **dadurch gekennzeichnet, dass** das Verdickungsmittel in einer Menge von 0,01 bis 6 Gew.-% bezüglich des Gesamtgewichts der Zusammensetzung vorhanden ist.

**38.** Zusammensetzung nach einem der vorhergehenden Ansprüche, **dadurch gekennzeichnet, dass** sie mindestens ein filmbildendes Polymer umfasst.

**39.** Zusammensetzung nach dem vorhergehenden Anspruch, **dadurch gekennzeichnet, dass** das filmbildende Polymer in einer Menge von 0,01 bis 4 Gew.-% bezüglich des Gesamtgewichts der Zusammensetzung vorhanden ist.

**40.** Zusammensetzung nach einem der vorhergehenden Ansprüche, **dadurch gekennzeichnet, dass** sie mindestens einen Füllstoff umfasst.

**41.** Zusammensetzung nach dem vorhergehenden Anspruch, **dadurch gekennzeichnet, dass** der Füllstoff in einem Verhältnis von 0,01 bis 60 Gew.-% bezüglich des Gesamtgewichts der Zusammensetzung vorhanden ist.

**42.** Schminkverfahren für die Haut, die Lippen und/oder der Hautanhangsgebilde, das das Aufbringen von mindestens einer Zusammensetzung nach einem der vorhergehenden Ansprüche auf die Haut, die Lippen und/oder die Hautanhangsgebilde umfasst.

**43.** Verwendung von mindestens einem photochromen Farbstoff, der eine Gesichtsfarbentoleranz $\Delta E$ von mindestens gleich 5 aufweist und ein Naphthopyran-Derivat der Formel (I) oder (II) ist, wie nach einem der Ansprüche 1 bis 8 definiert, in einer kosmetischen Zusammensetzung, insbesondere zum Schminken der Haut, der Hautanhangsgebilde, der Nägel und der Lippen, die mindestens einen zweiten Farbstoff umfasst, zur reversiblen Modifikation ihrer Farbe als Reaktion auf eine Bestrahlung, die UV-Strahlen enthält, wobei die Zusammensetzung 0,001 bis 20 Gew.-% des photochromen Farbstoffes bezüglich des Gesamtgewichts der Zusammensetzung umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 970689 A
- WO 02078665 A
- WO 9422850 A
- WO 9845281 A
- WO 0018755 A
- US 5362315 A
- US 5807497 A
- EP 1046692 A
- JP 09188830 A
- JP 10158450 A
- JP 10158541 A
- JP 07258460 A
- JP 05017710 A